# EUROPEAN PATENT APPLICATION

(11) **EP 4 372 015 A1**
(43) Date of publication of application: **22.05.2024**
(21) Application number: 22842057.6
(22) Date of filing: 08.07.2022
(51) Int. Cl.: C08F 20/56, B32B 15/08, C07C 233/09, C08J 5/24, C08L 33/26, C08L 101/00, C09J 7/30, H01B 3/44, H05K 1/03

(54) **NOVEL POLYMER, RESIN COMPOSITION INCLUDING SAME, AND MOLDED BODY THEREOF**

(30) Priority: 14.07.2021 JP 2021116330; 02.06.2022 JP 2022090373
(71) Applicant: Nippon Soda Co., Ltd., Tokyo 100-7010 (JP)
(72) Inventor: YAMATE, Taiki, Ichihara-shi, Chiba 290-0045 (JP); KAWANISHI, Takuya, Ichihara-shi, Chiba 290-0045 (JP)
(74) Representative: Mewburn Ellis LLP
(86) International application number: PCT/JP2022/027125
(87) International publication number: WO 2023/286713

(57) **Abstract**

An object of the present invention is to provide a polymer that has excellent heat resistance and dielectric properties and can be dissolved in an organic solvent at a high concentration, a resin composition containing the same, and a molded body thereof. The polymer of the present invention has a repeating unit derived from a polymerizable compound of the following formula (I) (In the formula, X¹ and X² each independently represent a C3 to C6 branched alkyl group, a C3 to C6 cyclic alkyl group, a C3 to C6 branched alkoxy group, or a C3 to C6 cyclic alkoxy group, n represents 0 or 1, Z¹ and Z² each independently represent a single bond or a C1 to C3 alkylene group, each R independently represents an organic group or a halogeno group, m1 and m2 each independently represent any integer of 0 to 4, and Y represents a polymerizable functional group).

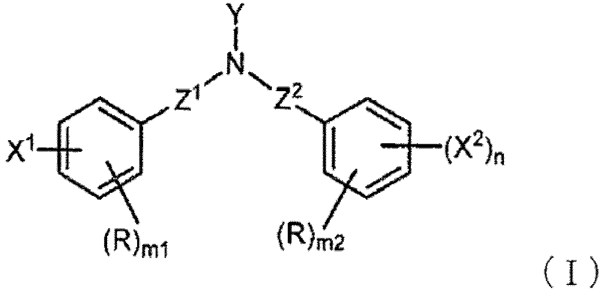

## Description

### Technical Field

The present invention relates to a polymer that has excellent heat resistance and dielectric properties and can be dissolved in an organic solvent at a high concentration, a resin composition containing the same, and a molded body thereof.

The present application claims priority over Japanese Patent Application No. 2021-116330 filed on July 14, 2021 and Japanese Patent Application No. 2022-90373 filed on June 2, 2022, and its contents are incorporated herein.

### Background Art

Various known resins are used to produce mobile communication devices such as mobile phones and smartphones, their base station equipment, network-related electronic devices such as servers and routers, and printed wiring boards included in large computers and the like.

In recent years, the above-described network-related electronic devices need to transmit and process large amounts of information at low loss and at high speed, and the electrical signals handled by the printed wiring boards of these products are also becoming increasingly high-frequency signals. The high-frequency electrical signals are easily attenuated, and thus it is necessary to further reduce transmission loss in printed wiring boards. Therefore, resins used in producing printed wiring boards are required to have a low dielectric constant and a low dielectric loss tangent.

In addition, the resins used in production of printed wiring boards are required to have high heat resistance (high glass transition point), be dissolved at a high concentration in common organic solvents for varnishes such as methyl ethyl ketone (MEK), toluene, and cyclohexanone.

For example, Patent Document 1 discloses a polyarylate resin.

### Prior Art Documents

### Patent Documents

[Patent Document 1] Japanese unexamined Patent Application Publication No. 2017-149940

### Summary of the Invention

### Object to be Solved by the Invention

However, since dielectric property and heat resistance are physical properties inversely proportional to solubility in organic solvents, it has been significantly difficult to satisfy all of the above physical properties.

An object of the present invention is to provide a polymer that has a low dielectric constant and a low dielectric loss tangent, has a high glass transition point, and exhibits high solubility in organic solvents and high compatibility with thermosetting resins, a resin composition containing the same, and a molded body thereof.

### Means to Solve the Object

As a result of intensive investigations, the inventors of the present invention have found that the above-described objects can be solved by a new polymer such as a specific polyacrylamide.

That is, the present invention relates to the following invention.
(1) A polymer having at least one repeating unit derived from a polymerizable compound of formula (I) 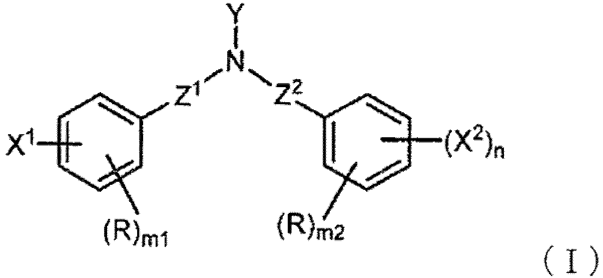 (wherein, X¹ and X² each independently represent a C3 to C6 branched alkyl group, a C3 to C6 cyclic alkyl group, a C3 to C6 branched alkoxy group, or a C3 to C6 cyclic alkoxy group, n represents 0 or 1, Z¹ and Z² each independently represent a single bond or a C1 to C3 alkylene group, each R independently represents an organic group or a halogeno group, m1 and m2 each independently represent any integer of 0 to 4, and Y represents a polymerizable functional group).
(2) The polymer according to (1), Y is an acryloyl group or a methacryloyl group.
(3) A resin composition comprising the polymer according to (1) .
(4) The resin composition according to (3), further comprising a resin other than the polymer according to (1).
(5) The resin composition according to (4), wherein the resin other than the polymer according to (1) is a thermosetting resin.
(6) The resin composition according to (5), wherein the thermosetting resin is an epoxy resin.
(7) The resin composition according to (6), further comprising an active ester-based compound as a curing agent.
(8) The resin composition according to (5), wherein the thermosetting resin is at least one or more selected from:
   a maleimide compound having one or more maleimide groups;
   a polyphenylene ether compound;
   a polybutadiene having a molar ratio of 1,2-bond structure to 1,4-bond structure of 80:20 to 100:0;
   a styrene-butadiene-styrene block copolymer (SBS) in which a molar ratio of 1,2-bond structure to 1,4-bond structure in a butadiene block is 80:20 to 100:0; and
   a polymer having, in a molecule, a repeating unit of formula (V) : 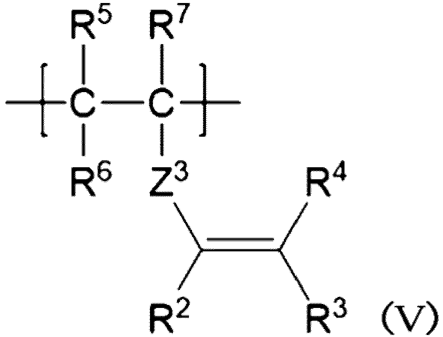
   (wherein Z³ represents a C6 to C12 arylene group, R² to R⁷ each independently represent a hydrogen atom or a C1 to C6 alkyl group).
(9) The resin composition according to (8), wherein a polyphenylene ether compound is a polyphenylene ether compound terminal-modified with a group of formula (VI), an acryloyl group, or a methacryloyl group, 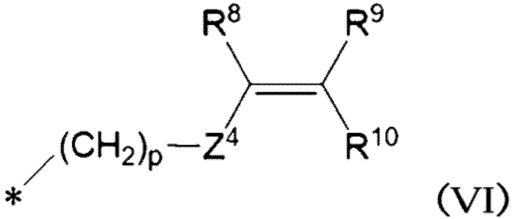 (wherein * represents a bonding position, p represents an integer of 0 to 10, Z⁴ represents a C6 to C12 arylene group, and R⁸ to R¹⁰ each independently represent a hydrogen atom or a C1 to C6 alkyl group).
(10) A molded body comprising a cured product of the resin composition according to any one of (3) to (9).
(11) The resin composition according to any one of (3) to (9), which is a resin composition for an insulating layer of printed wiring boards.
(12) A resin varnish comprising the resin composition according to any one of (3) to (9).
(13) A prepreg comprising a base material impregnated with the resin composition according to any one of (3) to (9).
(14) An adhesive film comprising a resin composition layer containing the resin composition according to any one of (3) to (9) on a support film.
(15) An insulator for printed wiring boards, consisting of a cured product of the prepreg according to (13).
(16) An insulator for printed wiring boards, consisting of a cured product of the adhesive film according to (14).
(17) A metal foil-attached laminate, comprising a layer consisting of the insulator for printed wiring boards according to (15) and a layer consisting of metal foil.
(18) A metal foil-attached laminate, comprising a layer consisting of the insulator for printed wiring boards according to (16) and a layer consisting of metal foil.
(19) A compound of formula (III) 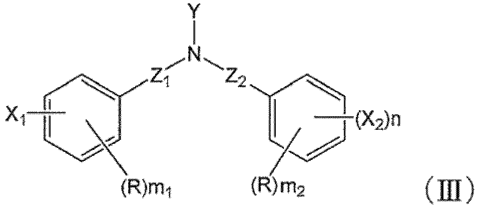 (wherein, X¹ and X² each independently represent a C3 to C6 branched alkyl group, a C3 to C6 cyclic alkyl group, a C3 to C6 branched alkoxy group, or a C3 to C6 cyclic alkoxy group, n represents 0 or 1, Z¹ and Z² each independently represent a single bond or a C1 to C3 alkylene group, each R independently represents an organic group or a halogeno group, m1 and m2 each independently represent any integer of 0 to 4, and Y represents a polymerizable functional group).
(20) The compound according to (19), wherein the compound of formula (III) is a compound of formula (IV) 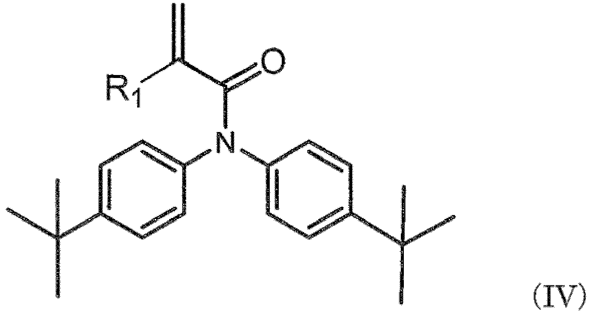 (wherein, R₁ represents a hydrogen atom or a methyl group).

### Effect of the Invention

The novel polymer of the present invention has a low dielectric constant and a low dielectric loss tangent, has a high glass transition point, and exhibits high solubility in organic solvents.

### Mode of Carrying Out the Invention

### 1. A polymer having a repeating unit derived from a polymerizable compound of formula (I)

The polymer of the present invention has a repeating unit derived from a polymerizable compound of formula (I).

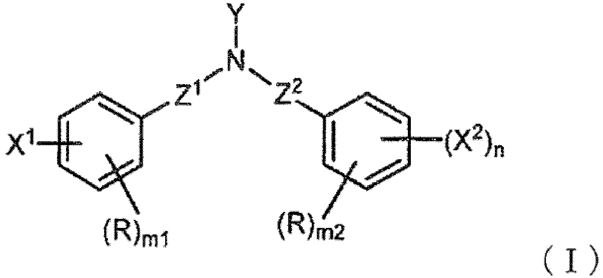

In formula (I),
X¹ and X² each independently represent a C3 to C6 branched alkyl group, a C3 to C6 cyclic alkyl group, a C3 to C6 branched alkoxy group, or a C3 to C6 cyclic alkoxy group.

As the "C3 to C6 branched alkyl group" in X¹ and X², an isopropyl group, a sec-butyl group, an isobutyl group, a tert-butyl group, a neopentyl group, an isopentyl group, a sec-pentyl group, a 1-ethylpropyl group, a tert-pentyl group, an isohexyl group, a 3-methylpentyl group, a 2,2-dimethylbutyl group, a 2,3-dimethylbutyl group, or the like may be exemplified.

As the "C3 to C6 cyclic alkyl group" in X¹ and X², a cyclopropyl group, a cyclobutyl group, a cyclopentyl group, a cyclohexyl group, or the like may be exemplified.

As the "C3 to C6 branched alkoxy group" in X¹ and X², an isopropyloxy group, a sec-butyloxy group, an isobutyloxy group, a tert-butyloxy group, a neopentyloxy group, an isopentyloxy group, a sec-pentyloxy group, a 1-ethylpropyloxy group, a tert-pentyloxy group, an isohexyloxy group, a 3-methylpentyloxy group, a 2,2-dimethylbutyloxy group, a 2,3-dimethylbutyloxy group, or the like may be exemplified.

As the "C3 to C6 cyclic alkoxy group" in X¹ and X², a cyclopropyloxy group, a cyclobutyloxy group, a cyclopentyloxy group, a cyclohexyloxy group, or the like may be exemplified.

X¹ and X² are preferably C3 to C6 branched alkyl groups, more preferably tert-butyl groups.

In formula (I), n represents 0 or 1.
n is preferably 1.

In formula (I), Z¹ and Z² each independently represent a single bond or a C1 to C3 alkylene group.

As the C1 to C3 alkylene group in Z¹ and Z², methylene, ethylene, propane-1,3-diyl, or the like may be exemplified.

Z¹ and Z² are each preferably a single bond.

In formula (I), each of R independently represents an organic group or a halogeno group.

The "organic group" is not particularly limited as long as it is chemically permissible and has the effects of the present invention. As the organic group, a C1 to C6 alkyl group such as a methyl group, an ethyl group, a n-propyl group, an i-propyl group, a n-butyl group, a s-butyl group, an i-butyl group, a t-butyl group, a n-pentyl group, and a n-hexyl group; a C6 to C10 aryl group such as a phenyl group and a naphthyl group; a C1 to C6 alkoxy group such as a methoxy group, an ethoxy group, a n-propoxy group, an i-propoxy group, a n-butoxy group, a s-butoxy group, an i-butoxy group, and a t-butoxy group; a C1 to C6 haloalkyl group such as a chloromethyl group, a chloroethyl group, a trifluoromethyl group, a 1,2-dichloro-n-propyl group, a 1-fluoro-n-butyl group, and a perfluoro-n-pentyl group; or the like may be exemplified.

As the "halogeno group," a fluoro group, a chloro group, a bromo group, an iodo group, or the like may be exemplified.

In formula (I), m1 and m2 each independently represent an integer of 0 to 4.

m1 and m2 are preferably 0.

In formula (I), Y represents a polymerizable functional group. As the polymerizable functional group, a group having a polymerizable carbon-carbon double bond such as a vinyl group, an allyl group, an acryloyl group, a methacryloyl group, a vinyloxycarbonyl group, a prop-1-en-2-yloxycarbonyl group, an allyloxycarbonyl group, or the like may be exemplified.

In the present invention, Y is preferably an acryloyl group or a methacryloyl group, and more preferably an acryloyl group.

The above formula (I) includes a compound of the following formula (II).

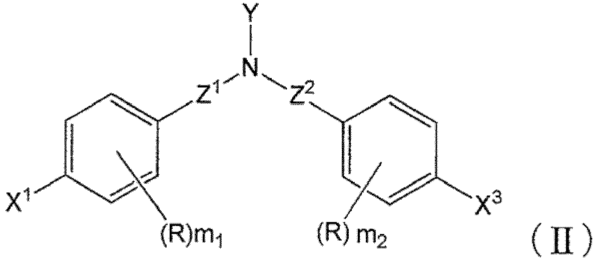

In formula (II), Y, Z¹, Z², X¹, R, m1, and m2 are the same as those explained in the formula (I).

In the formula (II), X³ represents a C3 to C6 branched alkyl group, a C3 to C6 cyclic alkyl group, a C3 to C6 branched alkoxy group, a C3 to C6 cyclic alkoxy group, a hydrogen atom, an organic group or a halogeno group. Among them, the C3 to C6 branched alkyl group, the C3 to C6 cyclic alkyl group, or the C3 to C6 branched alkoxy group is preferable, the C3 to C6 branched alkyl group is more preferable, and a tert-butyl group is particularly preferable. As the C3 to C6 branched alkyl group, the C3 to C6 cyclic alkyl group, the C3 to C6 branched alkoxy group, and the C3 to C6 cyclic alkoxy group in X³, the same as the C3 to C6 branched alkyl group, C3 to C6 cyclic alkyl group, C3 to C6 branched alkoxy group, and the C3 to C6 cyclic alkoxy group in X¹ and X² in the formula (I) may be exemplified.

Among the compounds of the formula (II), the compound of formula (IV) is particularly preferable.

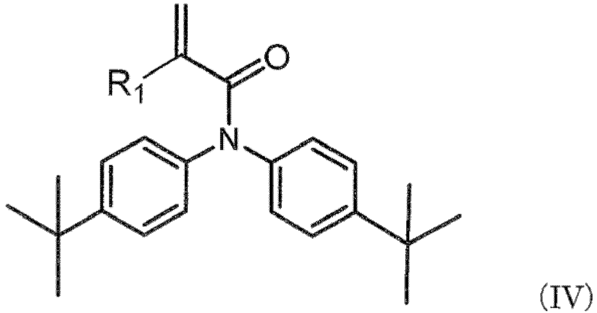

### (In the formula (IV), R₁ represents a hydrogen atom or a methyl group)

R₁ is preferably a hydrogen atom.

The polymer having a repeating unit derived from the polymerizable compound of the formula (I) used in the present invention is a polymer consisting of at least one repeating unit derived from the polymerizable compound of the following formula (I), or a polymer consisting of at least one of the repeating units and at least one repeating unit derived from another radically polymerizable compound.

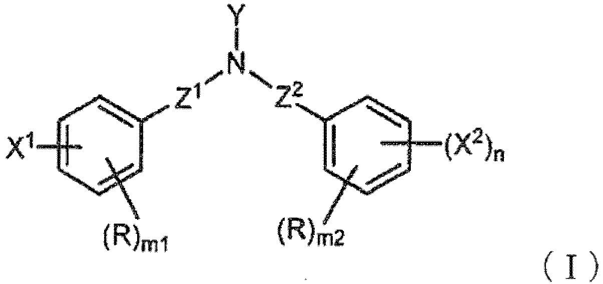

(In the formula (I), each symbol is as described above.)

That is, the polymers used in the present invention specifically include the following polymers.
I) A homopolymer consisting of one of the repeating unit derived from the polymerizable compound of the formula (I),
II) A copolymer consisting of two or more of the repeating units derived from the polymerizable compound of the formula (I),
III) A copolymer consisting of at least one repeating unit derived from the polymerizable compound of the formula (I) and at least one repeating unit derived from another radically polymerizable compound, and
IV) A copolymer consisting of two or more repeating units derived from the polymerizable compound of the formula (I) and at least one repeating unit derived from another radically polymerizable compound.

The polymer having the repeating unit derived from the polymerizable compound of the formula (I) is preferably I) a homopolymer consisting of one of the repeating unit derived from the polymerizable compound of the formula (I), or III) a copolymer consisting of at least one repeating unit derived from the polymerizable compound of the formula (I) and at least one repeating unit derived from another radically polymerizable compound, more preferably I) a homopolymer consisting of one of the repeating unit derived from the polymerizable compound of the formula (I).

Among the above polymers, the copolymers II) to IV) optionally have any of repeating units arranged randomly, alternately, or in a block arrangement.

In addition, in the case of the copolymer of the present invention, the molecular chain may be linear or branched. Examples of the branched chain include a branched chain formed by branching at one point (star type) and a branched chain formed by branching at multiple points (graft type).

The weight average molecular weight of the polymer of the present invention is not limited, and examples thereof include a copolymer with a weight average molecular weight within the range of 1,000 to 5,000,000, 5,000 to 1,000,000, 10,000 to 500,000, 10,000 to 200,000, or 20,000 to 100,000.

In addition, regarding to the molecular weight distribution (PDI) of the polymer according to the present invention, the ratio of the weight average molecular weight to the number average molecular weight (Mw/Mn) is preferably 1.0 to 5.0, more preferably 1.0 to 4.0, and most preferably 1.0 to 3.0.

The weight average molecular weight and number average molecular weight are values obtained by converting data measured by gel permeation chromatography (GPC) using THF as a solvent in terms of the molecular weight of polymethyl methacrylate used as a standard.

### (Other radically polymerizable compounds)

"Other radically polymerizable compounds" refers to radically polymerizable compounds other than compounds of formula (I), and may be selected as appropriate depending on the desired physical properties such as melting point, viscosity, or refractive index. Although not particularly limited, examples thereof include (meth)acrylic compounds, (meth)acrylonitrile, styrenic compounds, a vinyl-based compound, an olefin compound, an unsaturated carboxylic acid anhydride, and one or more of these may be used. Specifically, the same compounds as those exemplified in the component (1), radically polymerizable compound may be exemplified.

In the present invention, "(meth)acrylic" means "acrylic" and/or "methacrylic."

Particularly, as "other radically polymerizable compounds", (meth)acrylic compounds such as (meth)acrylate and (meth)acrylamide, unsaturated dicarboxylic acid anhydrides, and styrenic compounds are preferable.

Examples of the (meth)acrylate include alkyl (meth)acrylate, acrylate having an alicyclic hydrocarbon group, (meth)acrylate having an ether skeleton, (meth)acrylate having a cyclic ether skeleton, and (meth)acrylate having an aromatic group.

As the alkyl (meth)acrylate, methyl (meth)acrylate, ethyl (meth)acrylate, propyl (meth)acrylate, isopropyl (meth)acrylate, butyl (meth)acrylate, isobutyl (meth)acrylate, t-butyl (meth)acrylate, 2-ethylhexyl (meth)acrylate, isooctyl (meth)acrylate, lauryl (meth)acrylate, stearyl (meth)acrylate, isononyl (meth)acrylate, or the like may be exemplified.

As the (meth)acrylate having an alicyclic hydrocarbon group, cyclohexyl (meth)acrylate, dicyclopentanyl (meth)acrylate, dicyclopentenyl (meth)acrylate, dicyclopentenyloxyethyl (meth)acrylate, isobornyl (meth)acrylate, and methoxylated cyclodecatriene (meth)acrylate, or the like may be exemplified.

As the (meth)acrylate having an ether skeleton, 2-methoxyethyl (meth)acrylate, 2-ethoxyethyl (meth)acrylate, diethylene glycol monomethyl ether (meth)acrylate, or the like may be exemplified.

As the (meth)acrylate having a cyclic ether skeleton, glycidyl (meth)acrylate, furfuryl (meth)acrylate, tetrahydrofurfuryl (meth)acrylate, 3-ethyl-3-oxetanylmethyl (meth)acrylate, (2-methyl-ethyl-1,3-dioxolan-4-yl)methyl (meth)acrylate, cyclic trimethylolpropane formal (meth)acrylate, γ-butyrolactone (meth)acrylate, dioxolane (meth)acrylate, oxetane (meth)acrylate, (meth)acryloylmorpholine, or the like may be exemplified.

As the (meth) acrylate having an aromatic group, phenyl (meth)acrylate, benzyl (meth)acrylate, methylbenzyl (meth)acrylate, ethylbenzyl (meth)acrylate, propylbenzyl (meth)acrylate, methoxybenzyl (meth)acrylate, phenoxyethyl (meth)acrylate, chlorobenzyl (meth)acrylate, nonylphenoxy polyethylene glycol (meth)acrylate, or the like may be exemplified.

As the (meth)acrylamide, methyl (meth)acrylamide, dimethyl (meth)acrylamide, ethyl (meth)acrylamide, hydroxyethyl (meth)acrylamide, diethyl (meth)acrylamide, n-propyl (meth)acrylamide, dipropyl (meth)acrylamide, diisopropyl (meth)acrylamide, isopropylacrylamide, n-butyl (meth)acrylamide, or the like may be exemplified.

As the unsaturated dicarboxylic anhydride, maleic anhydride, citraconic anhydride, itaconic anhydride, or the like may be exemplified.

As the styrenic compounds, styrene, p-hydroxystyrene, p-chlorostyrene, p-bromostyrene, p-methylstyrene, p-methoxystyrene, p-t-butoxystyrene, p-t-butoxycarbonylstyrene, or the like may be exemplified.

As Other examples thereof, a vinyl compound such as vinyl acetate, vinyl chloride, vinylidene chloride, (meth)acrylonitrile, vinyl ether, acrolein, and divinylbenzene; and an olefin compound such as ethylene, propylene, butadiene, or the like may be exemplified.

### (Composition ratio of repeating unit)

In the polymer used in the present invention, the contents of the repeating unit derived from the polymerizable compound of the formula (I) and the repeating unit derived from another radically polymerizable isomer compound are not particularly limited, as long as the effect of the present invention is demonstrated. The repeating unit derived from the polymerizable compound of the formula (I) and the repeating units derived from another radically polymerizable isomer compound may be selected within the ranges of 99.5:0.5 to 0.5:99.5, 99.5:0.5 to 30:70, 99:1 to 30:70, 90:10 to 30:70, and 60:40 to 30:70, in terms of weight ratio.

### (Method for producing compound of formula (I))

The compound of the formula (I), which is a polymerizable compound used in the present invention, may be synthesized by the method described in Examples or other known methods.

For example, when Y in formula (I) is an acrylic group or a methacrylic group, the polymer may be produced by the following method.

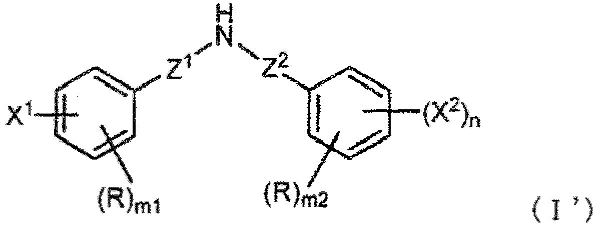

### (In the formula, X¹, X², n, Z¹, Z², R, m1, and m2 are the same as defined in formula (I))

A secondary amine and (meth)acrylic acid halide such as (meth)acrylic acid chloride of the above formula (I') are reacted in a solvent in the presence of a base.

As the solvent, an amide solvent such as N,N-dimethylformamide (DMF) and N,N-dimethylacetamide; an ether solvent such as tetrahydrofuran (THF), 1,2-dimethoxyethane, diethyl ether, and methyl cellosolve; an aromatic hydrocarbon such as benzene, toluene, xylene, chlorobenzene, dichlorobenzene, and benzonitrile; a saturated hydrocarbon such as pentane, hexane, octane, and cyclohexane; a halogenated hydrocarbon such as dichloromethane, chloroform, carbon tetrachloride, and 1,2-dichloroethane; a ketone solvent such as acetone, methyl ethyl ketone, methyl isobutyl ketone, and cyclohexanone; an ester solvent such as ethyl acetate, butyl acetate, and 2-methoxy-1-methylethyl acetate; or the like may be exemplified, and one or more of mixed solvents thereof may be used.

As the base, there may be used an aliphatic amine such as triethylamine and tributylamine; an aromatic amine such as pyridine, N-ethylpyridine, N,N-dimethylaniline, and N,N-dimethylaminopyridine; an organic base such as a metal alkoxide including sodium ethylate and sodium methylate; and an inorganic base such as a hydroxide of alkali metal or alkaline earth metal and a carbonate of alkali metal or alkaline earth metal, including lithium hydroxide, sodium hydroxide, potassium hydroxide, lithium carbonate, sodium carbonate, potassium carbonate, calcium hydroxide, magnesium hydroxide, calcium carbonate, magnesium carbonate, and sodium hydrogen carbonate.

The reaction temperature is -50°C to 200°C.

### (Polymerization method)

The polymer used in the present invention may be used without particular restrictions as long as it is a polymer obtained by polymerizing at least one polymerizable compound of formula (I), or it is a polymer obtained by polymerizing the compound thereof and another polymerizable compound. The polymerization reaction is not particularly limited, and may be any known method for synthesizing a polyacrylate, such as radical polymerization, anionic polymerization, cationic polymerization, ring-opening polymerization, coordination polymerization, and the like. An example is shown in Examples.

For example, when radical polymerizing a compound in which Y in formula (I) is an acrylic group or a methacrylic group with (meth)acrylate, polymerizable compounds of formula (I) and (meth)acrylate are heated or irradiated with light in a solvent in the presence of a radical polymerization initiator to perform a polymerization reaction.

The polymerization solvent is not particularly limited as long as it does not participate in the polymerization reaction and is compatible with the polymer, and specific examples thereof may include non-polar or low polar solvents such as an ether compound including diethyl ether, tetrahydrofuran (THF), and dioxane, trioxane; an ester compound including ethyl acetate; a ketone compound including methyl ethyl ketone and cyclohexanone; and an aliphatic, aromatic or cycloaliphatic hydrocarbon compound including hexane and toluene. These solvents may be used alone or as a mixed solvent of two or more.

As the radical polymerization initiator, azobisisobutyronitrile, azobis-2,4-dimethylvaleronitrile, azobiscyclohexanecarbonitrile, azobis-2-amidinopropane hydrochloride, potassium peroxodisulfate, ammonium peroxodisulfate, t-butyl hydroperoxide, di-t-butylcumene peroxide, acetyl peroxide, benzoyl peroxide, lauroyl peroxide, or the like may be exemplified.

### 2. Resin composition

The resin composition of the present invention contains a polymer (hereinafter also referred to as "component (A)") consisting of at least one repeating unit derived from the polymerizable compound of the above formula (I) .

The resin composition of the present invention may further contain, as a component other than (A) component, a curable resin such as an ionizing radiation-curable resin and a thermosetting resin (hereinafter also referred to as "(B) component") and other components.

### (1) Curable resin

As the curable resin, there may be used a (meth)acrylic resin, a vinyl resin, an allyl resin, a melamine resin, a urethane resin, an epoxy resin, a silicone resin, a polyester resin, a polyamic acid resin, a polyimide resin, a styrene maleic acid resin, a styrene maleic anhydride resin, a maleimide resin, and a cyanate resin. Herein, the term "ionizing radiation-curable resin" refers to a resin that is cured by irradiation with any ionizing radiation including all electromagnetic waves such as infrared rays, visible light, ultraviolet rays, X-rays, and electron beams.

The above ionizing radiation-curable resin and thermosetting resin are not particularly limited, and a monomer having a vinyl group, a (meth)acryloyl group, an epoxy group, an oxetanyl group, or a maleimide group, a prepolymer, an oligomer, a polymer, and the like may be used. Among these, a polyfunctional resin is preferably used. In the present invention, a "(meth)acryloyl group" means an "acryloyl group" and/or a "methacryloyl group".

Examples of thermosetting resins used as the resin composition of the present invention will be specifically explained below.

### 1) Epoxy resin (hereinafter also referred to as "(B-1) component")

As the epoxy resin, a bisphenol A type epoxy resin, a bisphenol F epoxy resin, a bisphenol S epoxy resin, a bisphenol AF epoxy resin, a dicyclopentadiene epoxy resin, a trisphenol epoxy resin, a naphthol novolac epoxy resin, a phenol novolac epoxy resin, an alicyclic epoxy resin having an ester skeleton, a tert-butyl-catechol epoxy resin, a naphthalene epoxy resin, a naphthol epoxy resin, an anthracene epoxy resin, a glycidylamine epoxy resin, a glycidyl ester epoxy resin, a cresol novolac epoxy resin, a biphenyl epoxy resin, a linear aliphatic epoxy resin, an epoxy resin with butadiene structure, an alicyclic epoxy resin, a heterocyclic epoxy resin, a spiro ring-containing epoxy resin, a cyclohexane dimethanol epoxy resin, a naphthylene ether epoxy resin, a trimethylol epoxy resin, a tetraphenylethane epoxy resin, or the like may be exemplified. The epoxy resin may be used alone or used by combination of two or more thereof.

The epoxy resin preferably contains an epoxy resin having two or more epoxy groups in one molecule. When the nonvolatile component of the epoxy resin accounts for 100% by mass, at least 50% by mass is preferably an epoxy resin having two or more epoxy groups in one molecule. Among them, it is preferable to include an epoxy resin that has two or more epoxy groups in one molecule and is liquid at a temperature of 20°C (hereinafter referred to as "liquid epoxy resin") and an epoxy resin that has three or more epoxy groups in one molecule and is solid at a temperature of 20°C (hereinafter referred to as "solid epoxy resin"). Using a liquid epoxy resin and a solid epoxy resin together as the epoxy resin may provide a resin composition having excellent flexibility. The breaking strength of the cured product of the resin composition is also improved.

As the liquid epoxy resin, a bisphenol A epoxy resin, a bisphenol F epoxy resin, a bisphenol AF epoxy resin, a naphthalene epoxy resin, a glycidyl ester epoxy resin, a phenol novolac epoxy resin, an alicyclic epoxy resin having an ester skeleton, an epoxy resin having a butadiene structure, a bisphenol A epoxy resin, a bisphenol F epoxy resin, a bisphenol AF epoxy resin, an alicyclic epoxy resin having an ester skeleton, and a naphthalene epoxy resin are more preferable; the bisphenol A epoxy resin and the bisphenol AF epoxy resin are more preferable; and the bisphenol AF epoxy resin is particularly preferable from the viewpoint of providing a cured product with excellent physical property balance.

As the solid epoxy resin, a naphthalene tetrafunctional epoxy resin, a cresol novolac epoxy resin, a dicyclopentadiene epoxy resin, a trisphenol epoxy resin, a naphthol epoxy resin, a biphenyl epoxy resin, a naphthylene ether epoxy resin, an anthracene epoxy resin, a bisphenol A epoxy resin, and a tetraphenylethane epoxy resin are preferable; the naphthalene tetrafunctional epoxy resin, the naphthol epoxy resin, and the biphenyl epoxy resin are more preferable; and the biphenyl epoxy resin is particularly preferable.

### (Curing agent)

When the above (B-1) is used as the thermosetting resin which is the (B) component, a curing agent may be contained.

As the curing agent for the epoxy resin, they are not limited, but a phenolic-based curing agent, a naphthol-based curing agent, an active ester-based curing agent, a benzoxazine-based curing agent, a cyanate ester-based curing agent, an acid anhydride-based curing agent, or the like may be exemplified, and from the viewpoint of lowering the dielectric loss tangent, the cyanate ester-based curing agent and the active ester-based curing agent are preferable. These may be used alone or used by combination of two or more thereof.

As the phenolic-based curing agent and naphthol-based curing agent, they are not particularly limited, but a phenol-based curing agent having a novolak structure and a naphthol-based curing agent having a novolac structure may be exemplified, and a phenol novolac resin, a triazine skeleton-containing phenol novolac resin, a naphthol novolac resin, a naphthol aralkyl resin, a triazine skeleton-containing naphthol resin, and a biphenylaralkyl phenolic resin are preferable.

As the active ester-based curing agent, a compound having two or more active ester groups in one molecule is preferable, and for example, compounds having two or more ester groups with high reaction activity in one molecule, such as phenol esters, thiophenol esters, N-hydroxyamine esters, and esters of heterocyclic hydroxy compounds, are preferably used. The active ester compound may be used alone or used by combination of two or more thereof.

From the viewpoint of improving heat resistance, there are preferable active ester compounds obtained by a condensation reaction between a carboxylic acid compound and/or a thiocarboxylic acid compound and a hydroxy compound and/or a thiol compound. Among them, there are more preferable active ester compounds obtained by reacting a carboxylic acid compound with one or more selected from a phenol compound, a naphthol compound, and a thiol compound, there are more preferable aromatic compounds having two or more active ester groups in one molecule, which are obtained by reacting a carboxylic acid compound with an aromatic compound having a phenolic hydroxyl group, and there is even more preferable aromatic compounds obtained by reacting a carboxylic acid compound having at least two or more carboxy groups in one molecule with an aromatic compound having a phenolic hydroxyl group, the aromatic compound having two or more active ester groups in one molecule. The active ester compound may be linear or branched.

As the carboxylic acid compound, an aliphatic carboxylic acid having 1 to 20 carbon atoms (preferably 2 to 10, more preferably 2 to 8) and an aromatic carboxylic acid having 7 to 20 carbon atoms (preferably 7 to 10) may be exemplified. As the aliphatic carboxylic acid, acetic acid, malonic acid, succinic acid, maleic acid, itaconic acid, or the like may be exemplified. As the aromatic carboxylic acid include benzoic acid, phthalic acid, isophthalic acid, terephthalic acid, pyromellitic acid, or the like may be exemplified. Among them, from the viewpoint of heat resistance, succinic acid, maleic acid, itaconic acid, phthalic acid, isophthalic acid, and terephthalic acid are preferable, and isophthalic acid and terephthalic acid are more preferable.

As the thiocarboxylic acid compound, they are not particularly limited, but thioacetic acid, thiobenzoic acid, or the like may be exemplified.

As the phenolic compound, a phenol compound having 6 to 40 carbon atoms (preferably 6 to 30, more preferably 6 to 23, even more preferably 6 to 22) may be exemplified, and as preferable specific examples thereof, hydroquinone, resorcinol, bisphenol A, bisphenol F, bisphenol S, phenolphthalin, methylated bisphenol A, methylated bisphenol F, methylated bisphenol S, phenol, o-cresol, m-cresol, p-cresol, catechol, dihydroxybenzophenone, trihydroxybenzophenone, tetrahydroxybenzophenone, phloroglucin, benzenetriol, dicyclopentadiene diphenol, or the like may be exemplified. As the phenol compound, a phenol novolak, a phosphorus atom-containing oligomer having a phenolic hydroxyl group described in Japanese unexamined Patent Application Publication No. 2013-40270 may also be used.

As the naphthol compound, a naphthol compound having 10 to 40 carbon atoms (preferably 10 to 30, more preferably 10 to 20) may be exemplified, and as preferable specific examples thereof, α-naphthol, β-naphthol, 1,5-dihydroxynaphthalene, 1,6-dihydroxynaphthalene, 2,6-dihydroxynaphthalene, or the like may be exemplified. A naphthol novolak may also be used as the naphthol compound.

Among them, there is
preferably bisphenol A, bisphenol F, bisphenol S, methylated bisphenol A, methylated bisphenol F, methylated bisphenol S, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, dicyclopentadiene diphenol, phenol novolak, or a phosphorus atom-containing oligomer having a phenolic hydroxyl group; there is more preferably catechol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzenetriol, dicyclopentadiene diphenol, phenol novolak, or a phosphorus atom-containing oligomer having a phenolic hydroxyl group; there is further more preferably 1,5-dihydroxynaphthalene, 1,6-dihydroxynaphthalene, 2,6-dihydroxynaphthalene, dihydroxybenzophenone, trihydroxybenzophenone, tetrahydroxybenzophenone, dicyclopentadiene diphenol, phenol novolak, or a phosphorus atom-containing oligomer having a phenolic hydroxyl group; there is even more preferably 1,5-dihydroxynaphthalene, 1,6-dihydroxynaphthalene, 2,6-dihydroxynaphthalene, dicyclopentadiene diphenol, phenol novolak, or a phosphorus atom-containing oligomer having a phenolic hydroxyl group; there is particularly preferably 1,5-dihydroxynaphthalene, 1,6-dihydroxynaphthalene, 2,6-dihydroxynaphthalene, dicyclopentadiene, diphenol, or a phosphorus atom-containing oligomer having a phenolic hydroxyl group; and the dicyclopentadiene diphenol is particularly preferable.

As the thiol compound, they are not particularly limited, but benzenedithiol, triazinedithiol, or the like may be exemplified.

As preferable specific examples of the active ester compound, an active ester compound containing dicyclopentadiene diphenol structure, an active ester compound containing naphthalene structure, an active ester compound including the acetylated product of phenol novolak, an active ester compound containing the benzoylated product of phenol novolak, an active ester compound obtained by reacting an aromatic carboxylic acid with a phosphorus atom-containing oligomer having a phenolic hydroxyl group may be exemplified, and among them, there is more preferable the active ester compound containing dicyclopentadiene type diphenol structure, the active ester compound containing naphthalene structure, and the active ester compound obtained by reacting an aromatic carboxylic acid with a phosphorus atom-containing oligomer having a phenolic hydroxyl group. In the present invention, "dicyclopentadiene diphenol structure" refers to a divalent structural unit consisting of phenylene-dicyclopentylene-phenylene.

As the active ester compound, active ester compounds disclosed in Japanese unexamined Patent Application Publication No. 2004-277460 and Japanese unexamined Patent Application Publication No. 2013-40270 may be used, and commercially available active ester compounds may also be used. As the commercially available active ester compound, EXB9451, EXB9460, EXB9460S, and HPC-8000-65T (manufactured by DIC Corporation) as the active ester compound containing a dicyclopentadiene diphenol structure; EXB9416-70BK (manufactured by DIC Corporation) as the active ester compound containing a naphthalene structure; DC808 (manufactured by Mitsubishi Chemical Corporation) as the active ester compound containing an acetylated product of phenol novolac; YLH1026 (manufactured by Mitsubishi Chemical Corporation) as the active ester compound containing a benzoylated phenol novolac; and EXB9050L-62M (DIC Corporation) as the phosphorus atom-containing active ester compound may be exemplified.

As the cyanate ester curing agent, they are not particularly limited, but a novolac (phenol novolac, alkylphenol novolac, and the like) cyanate ester-based curing agent, a dicyclopentadiene-based cyanate ester curing agent, a bisphenol (bisphenol A, bisphenol F, bisphenol S, and the like) cyanate ester-based curing agent, a prepolymer with these agents partially triazinized, or the like may be exemplified. The weight average molecular weight of the cyanate ester-based curing agent is not particularly limited, but is preferably from 500 to 4,500, more preferably from 600 to 3,000. As specific examples of the cyanate ester-based curing agent, a bifunctional cyanate resin such as bisphenol A dicyanate, polyphenol cyanate (oligo(3-methylene-1,5-phenylene cyanate), 4,4'-methylenebis(2,6-dimethylphenyl cyanate), 4,4'-ethylidene diphenyl dicyanate, hexafluorobisphenol A dicyanate, 2,2-bis(4-cyanato)phenylpropane, 1,1-bis(4-cyanatophenylmethane), bis(4-cyanato-3,5-dimethylphenyl)methane, 1,3-bis(4-cyanatophenyl-1-(methylethylidene))benzene, bis(4-cyanatophenyl)thioether, and bis(4-cyanatophenyl)ether; a polyfunctional cyanate resin derived from phenol novolak, cresol novolak, phenol resin containing dicyclopentadiene structure, and the like; a prepolymer with these cyanate resins partially triazinized; or the like may be exemplified. These may be used alone or used by combination of two or more thereof.

As the acid anhydride-based curing agent, they are not particularly limited, but phthalic anhydride, tetrahydrophthalic anhydride, hexahydrophthalic anhydride, methyltetrahydrophthalic anhydride, methylhexahydrophthalic anhydride, methylnadic anhydride, hydrogenated methyl nadic anhydride, trialkyltetrahydrophthalic anhydride, dodecenyl succinic anhydride, 5-(2,5-dioxotetrahydro-3-furanyl)-3-methyl-3-cyclohexene-1,2-dicarboxylic anhydride, trimellitic anhydride, pyromellitic anhydride, bensophenonetetracarboxylic dianhydride, biphenyltetracarboxylic dianhydride, naphthalenetetracarboxylic dianhydride, oxydiphthalic dianhydride, 3,3'-4,4'-diphenylsulfone tetracarboxylic dianhydride, 1,3,3a,4,5,9b-hexahydro-5-(tetrahydro-2,5-dioxo-3-furanyl)-naphtho[1,2-C]furan-1,3-dione, ethylene glycol bis(anhydrotrimellitate), a polymer acid anhydride such as styrene-maleic acid resin, which is obtained from a copolymerization of styrene and maleic acid, or the like may be exemplified.

### (Curing accelerator)

Further containing a curing accelerator may efficiently cure the epoxy resin and the curing agent. As the curing accelerator, they are not particularly limited, but an amine-based curing accelerator, a guanidine-based curing accelerator, an imidazole-based curing accelerator, a metal-based curing accelerator, a phosphonium-based curing accelerator, or the like may be exemplified. These may be used alone or used by combination of two or more thereof.

As the amine-based curing accelerator, they are not particularly limited, but a trialkylamine such as triethylamine and tributylamine, an amine compound such as 4-dimethylaminopyridine, benzyldimethylamine, 2,4,6-tris(dimethylaminomethyl)phenol, and 1,8-diazabicyclo(5,4,0)-undecene (hereinafter abbreviated as DBU), or the like may be exemplified. These may be used alone or used by combination of two or more thereof.

As the guanidine-based curing accelerator, they are not particularly limited, but dicyandiamide, 1-methylguanidine, 1-ethylguanidine, 1-cyclohexylguanidine, 1-phenylguanidine, 1-(o-tolyl)guanidine, dimethylguanidine, diphenylguanidine, trimethylguanidine, tetramethylguanidine, pentamethylguanidine, 1,5,7-triazabicyclo[4.4.0]dec-5-ene, 7-methyl-1,5,7-triazabicyclo[4.4.0]dec-5-en, 1-methylbiguanide, 1-ethylbiguanide, 1-n-butylbiguanide, 1-n-octadecyl biguanide, 1,1-dimethyl biguanide, 1,1-diethyl biguanide, 1-cyclohexyl biguanide, 1-allyl biguanide, 1-phenyl biguanide, 1-(o-tolyl) biguanide, or the like may be exemplified. These may be used alone or used by combination of two or more thereof.

As the imidazole-based curing accelerator, they are not particularly limited, but an imidazole compound such as 2-methylimidazole, 2-undecylimidazole, 2-heptadecyl imidazole, 1,2-dimethylimidazole, 2-ethyl-4-methylimidazole, 1,2-dimethylimidazole, 2-ethyl-4-methylimidazole, 2-phenylimidazole, 2-phenyl-4-methylimidazole, 1-benzyl-2-methylimidazole, 1-benzyl-2-phenylimidazole, 1-cyanoethyl-2-methylimidazole, 1-cyanoethyl-2-undecylimidazole, 1-cyanoethyl-2-ethyl-4-methylimidazole, 1-cyanoethyl-2-phenylimidazole, 1-cyanoethyl-2-undecylimidazolium trimellitate, 1-cyanoethyl-2-phenylimidazolium trimellitate, 2,4-diamino-6-[2'-methylimidazolyl-(1')]-ethyl-s-triazine, 2,4-diamino-6-[2'-undecylimidazolyl-(1')]-ethyl-s-triazine, 2,4-diamino-6-[2'-ethyl-4'-methylimidazolyl-(1')]-ethyl-s-triazine, 2,4-diamino-6-[2'-methylimidazolyl-(1')]-ethyls-triazine isocyanuric acid adduct, 2-phenylimidazole isocyanuric acid adduct, 2-phenyl-4,5-dihydroxymethylimidazole, 2-phenyl-4-methyl-5hydroxymethylimidazole, 2,3-dihydro-1H-pyrrolo[1,2-a]benzimidazole, 1-dodecyl-2-methyl-3-benzylimidazolium chloride, 2-methylimidazoline, 2-phenylimidazoline, and an adduct of an imidazole compound and an epoxy resin may be exemplified. These may be used alone or used by combination of two or more thereof.

As the metal-based curing accelerator, they are not particularly limited, an organometallic complex or organometallic salt of a metal such as cobalt, copper, zinc, iron, nickel, manganese, and tin may be exemplified. As specific examples of the organometallic complex, an organic cobalt complex such as cobalt (II) acetylacetonate and cobalt (III) acetylacetonate; an organocopper complex such as copper (II) acetylacetonate; an organozinc complex such as zinc (II) acetylacetonate; an organic iron complex such as iron (III) acetylacetonate; an organic nickel complex such as nickel (II) acetylacetonate; an organic manganese complex such as manganese (II) acetylacetonate; or the like may be exemplified. As the organic metal salt, zinc octylate, tin octylate, zinc naphthenate, cobalt naphthenate, tin stearate, zinc stearate, or the like may be exemplified. These may be used alone or used by combination of two or more thereof.

As the phosphonium-based curing accelerator (phosphorus-based curing accelerator), they are not particularly limited, but an organic phosphin such as triphenylphosphine, diphenyl(alkylphenyl)phosphine, tris(alkylphenyl)phosphine, tris(alkoxyphenyl)phosphine, tris(alkylalkoxyphenyl)phosphine, tris(dialkylphenyl)phosphine, tris(trialkylphenyl)phosphine, tris(tetraalkylphenyl)phosphine, tris(dialkoxyphenyl)phosphine, tris(trialkoxyphenyl)phosphine, tris(tetraalkoxyphenyl)phosphine, trialkylphosphine, dialkylarylphosphine, and alkyldiarylphosphine; a complex of organic phosphines and organic borons such as a phosphonium borate compound, tetraphenylphosphonium tetraphenylborate, and n-butylphosphonium tetraphenylborate; and an aromatic phosphonium salt, an adduct of tertiary phosphine and quinones such as (4-methylphenyl)triphenylphosphonium thiocyanate, tetraphenylphosphonium thiocyanate, butyltriphenylphosphonium thiocyanate, and tetrabutylphosphonium decanoate, or the like may be exemplified.

As the tertiary phosphine, they are not particularly limited, but tri-n-butylphosphine, dibutylphenylphosphine, butyldiphenylphosphine, ethyldiphenylphosphine, triphenylphosphine, tris(4-methylphenyl)phosphine, tris(4-methoxyphenyl) phosphine, or the like may be exemplified. In addition, as the quinones, o-benzoquinone, p-benzoquinone, diphenoquinone, 1,4-naphthoquinone, anthraquinone, or the like may be exemplified. These may be used alone or used by combination of two or more thereof.

The curing accelerator used in the resin composition of the present invention is preferably an amine-based curing accelerator.

### 2) Maleimide compound having one or more maleimide groups (hereinafter also referred to as "(B-2) component")

The maleimide compound used in the present invention is not particularly limited as long as it has one or more maleimide groups in the molecule. As specific examples thereof, N-phenylmaleimide, N-hydroxyphenylmaleimide, bis(4-maleimidophenyl)methane, 2,2-bis{4-(4-maleimidophenoxy)-phenyl}propane, bis(3,5-dimethyl-4-maleimidophenyl)methane, bis(3-ethyl-5-methyl-4-maleimidophenyl)methane, bis(3,5-diethyl-4-maleimidophenyl)methane, polytetramethylene oxide-bis(4-maleimidobenzoate), 2,2-bis[4-(4-maleimidophenoxy)phenyl]propane, a maleimide compound of the following formula (VII), a maleimide compound of the following formula (IX), a prepolymer of these maleimide compounds, and a prepolymer of a maleimide compound and an amine compound may be exemplified. These may be used alone or in a suitable mixture of two or more.

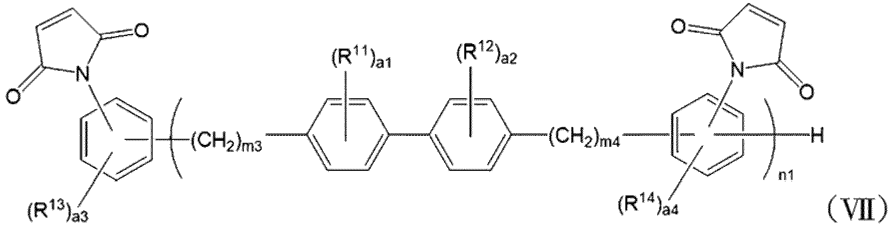

In formula (VII),
R¹¹ and R¹² each independently represent a halogeno group, a C1 to C6 alkyl group, a hydroxyl group, a C1 to C6 alkoxy group, a formyl group, a carboxyl group, a C1 to C6 alkoxycarbonyl group, a C6 to C10 aryl group, an amino group, a C1 to C6 alkyl group-substituted amino group, cyano group, or nitro group,
a1 and a2 each independently represent an integer from 0 to 4, when a1 is 2 or more, R¹¹ may be the same or different from each other, and when a2 is 2 or more, R¹² may be the same or different from each other,
m3 and m4 each independently represent an integer from 1 to 10,
R¹³ and R¹⁴ are each independently a C1 to C6 alkyl group or a C6 to C10 aryl group,
a3 represents an integer from 0 to 3, and when a3 is 2 or more, R¹³ may be the same or different from each other,
a4 represents an integer from 0 to 2, and when a4 is 2, R¹⁴ may be the same or different from each other, and
n1 represents an integer from 1 to 100.

As the "halogeno group" for R¹¹ and R¹², a fluoro group, a chloro group, a bromo group, an iodo group, or the like may be exemplified.

The "C1 to C6 alkyl group" in R¹¹ and R¹² may be linear or branched. As the C1 to C6 alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, a 2,2-dimethylpropyl group, and an i-hexyl group may be exemplified.

As the "C1 to C6 alkoxy group" in R¹¹ and R¹², a methoxy group, an ethoxy group, a n-propoxy group, an i-propoxy group, a n-butoxy group, a s-butoxy group, an i-butoxy group, a t-butoxy group, and the like may be exemplified.

As the "C1 to C6 alkoxycarbonyl group" in R¹¹ and R¹², a methoxycarbonyl group, an ethoxycarbonyl group, a n-propoxycarbonyl group, an i-propoxycarbonyl group, a n-butoxycarbonyl group, a t-butoxycarbonyl group, and the like may be exemplified.

As the "C6 to C10 aryl group" in R¹¹ and R¹², a phenyl group, a naphthyl group, and the like may be exemplified.

The "C1 to C6 alkyl group-substituted amino group" in R¹¹ and R¹² may be either mono-substituted or di-substituted. As the "C1 to C6 alkyl group-substituted amino group", a methylamino group, a dimethylamino group, an ethylamino group, a diethylamino group, a methylethylamino group, and the like may be exemplified.

a1 and a2 are preferably 0.

m3 and m4 are preferably 1 to 6, more preferably 1 to 3, even more preferably 1 to 2, and 1 is particularly preferable.

As the C1 to C6 alkyl group and C6 to C10 aryl group in R¹³ and R¹⁴, the same examples as those exemplified in R¹¹ and R¹² may be exemplified.

a3 and a4 are preferably 0.

n1 is preferably from 1 to 50, more preferably from 1 to 20, even more preferably from 1 to 5.

As the compound of formula (VII), specifically, a compound of formula (VIII) may be exemplified.

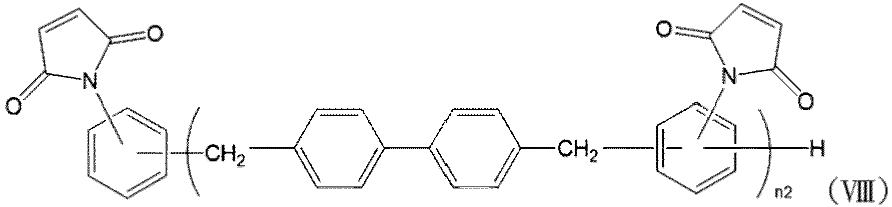

In formula (VIII), n2 represents any integer of 1 to 100.

n2 is preferably from 1 to 50, more preferably from 1 to 20, even more preferably from 1 to 5. 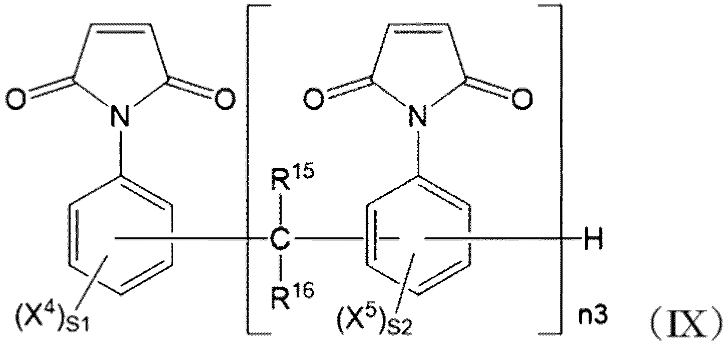 In formula (IX), R¹⁵ and R¹⁶ each independently represent a hydrogen atom or a C1 to C6 alkyl group, n3 represents any integer from 1 to 10, X⁴ and X⁵ each independently represent a C1 to C6 alkyl group, S1 and S2 each independently represent any integer from 0 to 2, when S1 is 2, X⁴ may be the same or different from each other, and when S2 is 2, X⁵ may be the same or different from each other.

As the "C1 to C6 alkyl group" in R¹⁵ and R¹⁶, the same examples as those exemplified in R¹¹ and R¹² may be exemplified.

R¹⁵ and R¹⁶ are preferably a hydrogen atom or a methyl group, and more preferably a hydrogen atom.

n3 is preferably an integer from 1 to 7, more preferably an integer from 1 to 3, further preferably 1.

As the "C1 to C6 alkyl group" in X⁴ and X⁵, the same examples as those exemplified in R¹¹ and R¹² may be exemplified.

X⁴ and X⁵ are preferably a methyl group or an ethyl group.

The compound of formula (IX) is preferably 4,4'-diphenylmethane bismaleimide or bis(3-ethyl-5-methyl-4-maleimidophenyl)methane.

The maleimide compound used in the present invention is preferably a maleimide compound of formula (VII) or a maleimide compound of formula (IX), more preferably a compound of formula (VIII) or a maleimide compound of formula (IX).

Commercially available maleimide compounds may be used. As the commercially available product, for example, MIR-3000 (manufactured by Nippon Kayaku Co., Ltd.), MIR-3000-70MT (manufactured by Nippon Kayaku Co., Ltd.), BMI (manufactured by K•I Chemical Industry Co., Ltd.), BMI-70 (manufactured by K•I Chemical Industry Co., Ltd.), BMI-80 (manufactured by K•I Chemical Industry Co., Ltd.), and the like may be exemplified.

### 3) Polyphenylene ether compound (hereinafter also referred to as "(B-3) component")

The polyphenylene ether compound used in the present invention is not particularly limited as long as it is a polymer having a repeating unit of the following formula (X) .

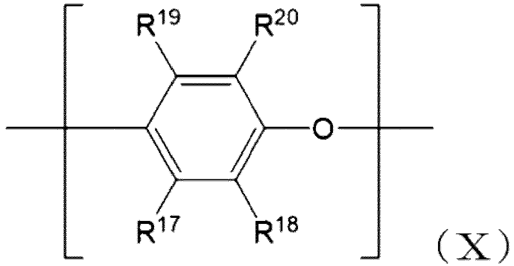

In formula (X), R¹⁷ to R²⁰ each independently represent a hydrogen atom, a C1 to C6 alkyl group, a C2 to C6 alkenyl group, a C2 to C6 alkynyl group, a C1 to C6 alkylcarbonyl group, or a C2 to C6 alkenylcarbonyl group.

As the "C1 to C6 alkyl group" in R¹⁷ to R²⁰, the same examples as those exemplified in R¹¹ and R¹² may be exemplified.

As the "C2 to C6 alkenyl group" in R¹⁷ to R²⁰, a vinyl group, an allyl group, and the like may be exemplified.

As the "C2 to C6 alkynyl group" in R¹⁷ to R²⁰, ethynyl, 2-propynyl, and the like may be exemplified.

As the "C1 to C6 alkylcarbonyl group" in R¹⁷ to R²⁰, an acetyl group and the like may be exemplified.

As the "C2 to C6 alkenylcarbonyl group" in R¹⁷ to R²⁰, an acryloyl group, a methacryloyl group, and the like may be exemplified.

R¹⁷ to R²⁰ are preferably a hydrogen atom, a C1 to C6 alkyl group, or a C2 to C6 alkenyl group, more preferably a hydrogen atom, a methyl group, or an allyl group, and more preferably a hydrogen atom or a methyl group.

The polyphenylene ether compound used in the present invention optionally has its terminal modified. As the polyphenylene ether compound with its terminal modified, a polyphenylene ether compound with its terminal modified with a hydroxyl group, a polyphenylene ether compound modified with a substituent having a carbon-carbon unsaturated double bond, and the like may be exemplified.

As the substituent having the carbon-carbon unsaturated double bond, a group of formula (VI), an acryloyl group, or methacryloyl group may be exemplified. 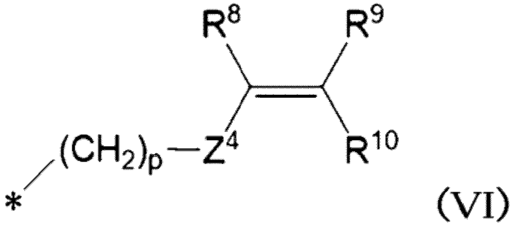 In formula (VI), * represents a bonding position, p represents an integer of 0 to 10, Z⁴ represents an arylene group, and R⁸ to R¹⁰ each independently represent a hydrogen atom or a C1 to C6 alkyl group.

As the C6 to C12 arylene group in Z⁴, a phenylene group may be exemplified.

As the C1 to C6 alkyl group in R⁸ to R¹⁰, the same examples as those exemplified in R¹¹ and R¹² may be exemplified.

As the group of formula (VI), specifically, a group of formula (VI-1) or formula (VI-2) may be exemplified.

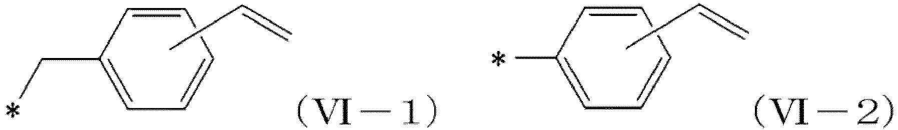

In formula (VI-1) and formula (VI-2), * represents the bonding position.

As the polyphenylene ether compound modified with a substituent having a carbon-carbon unsaturated double bond, specifically, a compound of formula (3) or formula (4) may be exemplified. 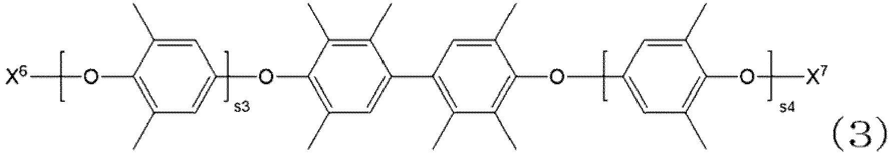 In formula (3), X⁶ and X⁷ each independently represent a group of formula (VI), an acryloyl group, or a methacryloyl group, and s3 and s4 each independently represent an integer from 0 to 20. The groups of formula (VI) in X⁶ and X⁷ are as described above. 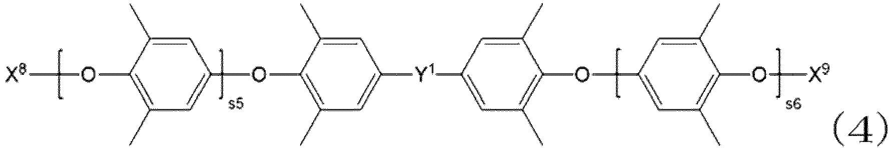 In formula (4), X⁸ and X⁹ each independently represent a group of formula (VI), an acryloyl group, or a methacryloyl group, s5 and s6 each independently represent an integer from 0 to 20, and Y¹ represents a C1 to C6 alkylene group. The groups of the formula (VI) in X⁸ and X⁹ are as described above. As the "C1 to C6 alkylene group" in Y¹, a methylene group, an ethylene group, a methylmethylene group, a dimethylmethylene group, and the like may be exemplified.

The number average molecular weight (Mn) of the polyphenylene ether compound used is not particularly limited, but 1,000 to 7,000, 1,000 to 5,000, and 1,000 to 3,000 may be exemplified. The number average molecular weight (Mn) is a value obtained by converting data measured by gel permeation chromatography (GPC) using tetrahydrofuran as a solvent in terms of the molecular weight of standard polystyrene.

In addition, as the intrinsic viscosity of the polyphenylene ether compound used in the present invention, 0.03 to 0.12 dl/g, 0.04 to 0.11 dl/g, 0.06 to 0.095 dl/g, and the like may be exemplified. Intrinsic viscosity is the intrinsic viscosity measured in methylene chloride at 25°C. More specifically, intrinsic viscosity is a value measured with a viscometer using a 0.18 g/45 ml methylene chloride solution (liquid temperature: 25°C).

As the polyphenylene ether compound used in the present invention, known ones or commercially available products may be used. As the commercially available products, SA90 (manufactured by SABIC), SA9000 (manufactured by SABIC), and OPE-2st (manufactured by Mitsubishi Gas Chemical Company, Inc.) may be exemplified.

When synthesizing, synthesis may be performed by the method described in WO2014/203511 and the like or a method analogous thereto.

### 4) Polybutadiene having a molar ratio of 1,2-bond structure to 1,4-bond structure of 80:20 to 100:0 (hereinafter also referred to as "(B-4) component")

The polybutadiene used in the present invention consists of only 1,2-bond structure of formula (1), or 1,2-bond structure of formula (1) and 1,4-bond structure of formula (2).

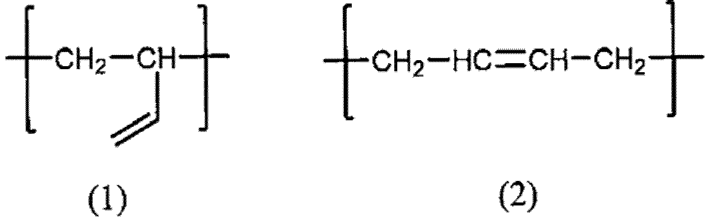

The ratio of the 1,2-bond structure of formula (1) to the 1,4-bond structure of formula (2) included in polybutadiene is not particularly limited, but among all the repeating units of polybutadiene, the molar ratio of the 1,2-bond structure of formula (I) is preferably 80:20 to 100:0.

The molecular weight of the polybutadiene used is not particularly limited, but a weight average molecular weight (Mw) of 500 to 10,000, 500 to 8,000, 500 to 6,000, or 500 to 5,000 may be selected. The weight average molecular weight (Mw) is a value obtained by converting data measured by gel permeation chromatography (GPC) using tetrahydrofuran as a solvent in terms of the molecular weight of standard polystyrene.

The polybutadiene may be a polybutadiene with its main chain and terminals modified, or a polybutadiene with its main chain and terminals unmodified. Among them, from the viewpoint of obtaining a cured product having high insulation properties, it is preferable to use the polybutadiene with its main chain and terminals unmodified.

As the polybutadiene, commercially available products may be used. As the commercially available polybutadiene, NISSO-PB B-1000 (manufactured by Nippon Soda Co., Ltd.), NISSO-PB B-2000 (manufactured by Nippon Soda Co., Ltd.), NISSO-PB B-3000 (manufactured by Nippon Soda Co., Ltd.), and the like may be exemplified. These polybutadienes may be used alone or used by combination of two or more thereof.

### 5) Styrene-butadiene-styrene block copolymer (SBS) in which molar ratio of 1,2-bond structure to 1,4-bond structure in butadiene block is 80:20 to 100:0 (hereinafter referred to as "(B-5) component")

The styrene block in the styrene-butadiene-styrene block copolymer (SBS) used in the present invention is a block obtained by polymerizing styrene, and the butadiene block is a block obtained by polymerizing butadiene. The butadiene block consists of only a 1,2-bond structure of formula (1), or a 1,2-bond structure of formula (1) and a 1,4-bond structure of formula (2).

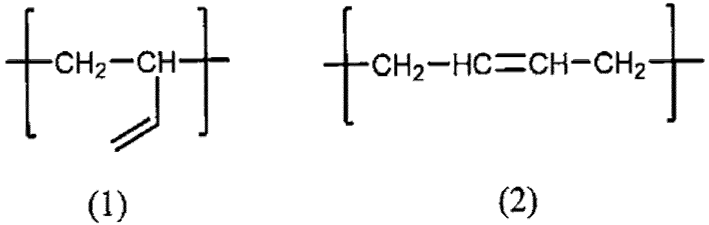

The molar ratio of the 1,2-bond structure of formula (1) to the 1,4-bond structure of formula (2) included in the styrene-butadiene-styrene block copolymer used in the present invention is 80:20 to 100:0.

The weight ratio of the styrene block and butadiene block in the styrene-butadiene-styrene block copolymer is not particularly limited, but 10:90 to 80:20, 10:90 to 70:30, 10:90 to 60:40, 20:80 to 80:20, 30:70 to 80:20, and 40:60 to 80:20 may be exemplified.

The weight average molecular weight (Mw) of the styrene-butadiene-styrene block copolymer is not particularly limited, but 2,000 to 100,000, 2,000 to 80,000, 2,000 to 60,000, and the like may be exemplified. The molecular weight distribution (Mw/Mn) of the styrene-butadiene-styrene block copolymer is not particularly limited, but 1.00 to 3.00, 1.00 to 2.00, and the like may be exemplified. The weight average molecular weight (Mw) and molecular weight distribution (Mw/Mn) are measured by gel permeation chromatography (GPC) using polystyrene as a standard substance. The measurement conditions are a mobile phase of THF (tetrahydrofuran), a mobile phase flow rate of 1 mL/min, a column temperature of 40°C, a sample injection amount of 40 µL, and a sample concentration of 2% by weight.

The method for producing the styrene-butadiene-styrene block copolymer used is not particularly limited. For example, production may be performed by the methods described in Japanese unexamined Patent Application Publication No. 6-192502, Japanese unexamined Patent Application Publication (Translation of PCT Application) No. 2000-514122, Japanese unexamined Patent Application Publication No. 2007-302901, and WO2021/024679, and the methods analogous thereto.

### 6) Polymer having repeating unit of formula (V) in molecule (hereinafter also referred to as "(B-6) component")

The resin composition of the present invention may contain a polymer having a repeating unit of formula (V) in the molecule.

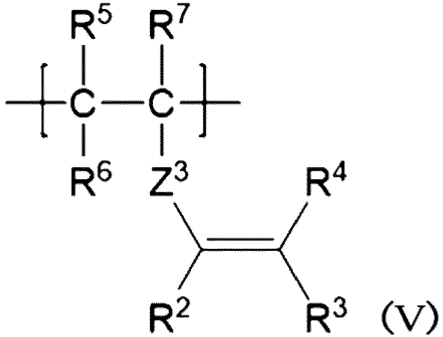

In formula (V), Z³ represents a C6 to C12 arylene group, and R² to R⁷ each independently represent a hydrogen atom or a C1 to C6 alkyl group.

As the "C6 to C12 arylene group" in Z³, the same ones as exemplified in Z⁴ may be exemplified.

As the "C1 to C6 alkyl group" in R² to R⁷, same examples as those exemplified in R¹¹ and R¹² may be exemplified.

As formula (V), specifically, formula (V-1) may be exemplified.

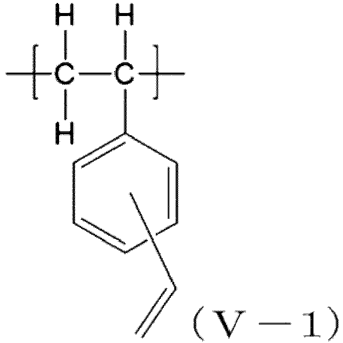

The polymer having a repeating unit of formula (V) in the molecule may be a polymer further having a structural unit of formula (5) in the molecule.

In addition, the polymer optionally has a repeating unit other than the repeating unit of formula (V) and the repeating unit of formula (5).

That is, the polymer having a repeating unit of formula (V) in the molecule of (B-6) component includes the following polymers.
I) A polymer consisting of one or more repeating units of formula (V)
II) A polymer consisting of one or more repeating units of formula (V) and one or more repeating units of formula (5)
III) A polymer consisting of one or more repeating units of formula (V), one or more repeating units of formula (5), and repeating units other than these.

Among these polymers, the copolymer may be a random copolymer or a block copolymer.

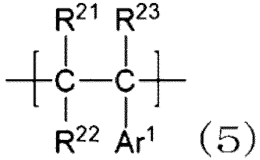

In formula (5), R²¹ to R²³ each independently represent a hydrogen atom or a C1 to C6 alkyl group, and Ar¹ represents a C6 to C12 aryl group substituted or unsubstituted with a C1 to C6 alkyl group.

As the "C1 to C6 alkyl group" in R²¹ to R²³, same examples as those exemplified in R¹¹ and R¹² may be exemplified.

As the "C6 to C12 aryl group" in Ar¹, a phenyl group, a naphthyl group, a biphenyl group, and the like may be exemplified.

As the "C1 to C6 alkyl group" that is a substituent on Ar¹, the same ones as exemplified in R¹¹ and R¹² may be exemplified.

As the formula (5), specifically, formula (5-1) and formula (5-2) may be exemplified.

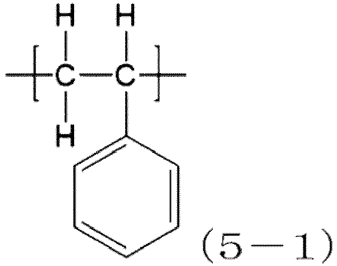

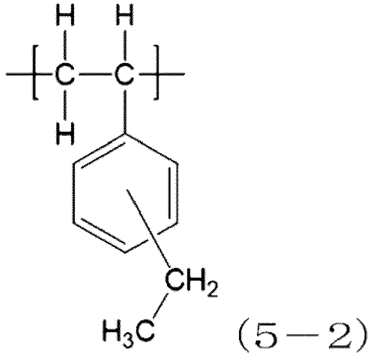

As specific examples of the polymer having a repeating unit of formula (V) in the molecule, a polymer including a repeating unit of formula (V-1) in the molecule, and further including at least one of a repeating unit of formula (5-1) and a repeating unit of formula (5-2) may be exemplified. This polymer may be a block copolymer or a random copolymer.

The weight average molecular weight of the polymer having a repeating unit of formula (V) in the molecule is preferably 1,200 to 40,000, more preferably 1,200 to 35,000. In addition, if the weight average molecular weight is too high, the moldability and the like tend to decrease. Therefore, the weight average molecular weight of the resin composition within the above range will provide excellent heat resistance and moldability. The weight average molecular weight herein may be one measured by general molecular weight measurement, and specifically, a value measured using gel permeation chromatography (GPC) and the like may be exemplified.

In the polymer having the repeating unit of formula (V) in the molecule, when the total of the repeating unit in the polymer having the repeating unit of formula (V) in the molecule is 100 mol%, the molar content of the repeating unit of formula (V) is preferably 2 to 95 mol%, more preferably 8 to 81 mol%.

In addition, when the polymer having the repeating unit of formula (V) in the molecule is a polymer having the repeating unit of formula (V) and the repeating unit of formula (5) in the molecule, the molar content of the repeating unit of the formula (V) is preferably 2 to 95 mol%, more preferably 8 to 81 mol%, and the molar content of the repeating unit of the formula (5) is preferably 5 to 98 mol%, more preferably 19 to 92 mol%.

### 7) (Meth)acrylate resin (hereinafter also referred to as "(B-7) component")

As the (meth)acrylate resin, a monofunctional (meth)acrylate monomer or (meth)acrylate oligomer may be used. Among them, it is preferable to include a polyfunctional (meth)acrylate having two or more polymerizable unsaturated groups.

The monofunctional (meth)acrylate monomer is a monomer that has one (meth) acrylate group in the molecule, and includes alkyl (meth)acrylate, acrylate having an alicyclic hydrocarbon group, (meth)acrylate having an ether skeleton, (meth)acrylate having a cyclic ether skeleton, (meth)acrylate having an aromatic group, and the like.

Examples of the alkyl (meth)acrylate include methyl (meth)acrylate, ethyl (meth)acrylate, propyl (meth)acrylate, isopropyl (meth)acrylate, butyl (meth)acrylate, isobutyl (meth)acrylate, t-butyl (meth)acrylate, 2-ethylhexyl (meth)acrylate, isooctyl (meth)acrylate, lauryl (meth)acrylate, stearyl (meth)acrylate, and isononyl (meth)acrylate.

Examples of the (meth)acrylate having an alicyclic hydrocarbon group include cyclohexyl (meth)acrylate, dicyclopentanyl (meth)acrylate, dicyclopentenyl (meth)acrylate, dicyclopentenyloxyethyl (meth)acrylate, isobornyl (meth)acrylate, and methoxylated cyclodecatriene (meth)acrylate.

Examples of the (meth)acrylate having an ether skeleton include 2-methoxyethyl (meth)acrylate, 2-ethoxyethyl (meth)acrylate, and diethylene glycol monomethyl ether (meth)acrylate.

Examples of the (meth)acrylate having a cyclic ether skeleton include glycidyl (meth)acrylate, furfuryl (meth)acrylate, tetrahydrofurfuryl (meth)acrylate, 3-ethyl-3-oxetanylmethyl (meth)acrylate, (2-methyl-ethyl-1,3-dioxolan-4-yl)methyl (meth)acrylate, cyclic trimethylolpropane formal (meth)acrylate, γ-butyrolactone (meth)acrylate, dioxolane (meth)acrylate, oxetane (meth)acrylate, and (meth)acryloylmorpholine.

Examples of the (meth)acrylate having an aromatic group include phenyl (meth)acrylate, benzyl (meth)acrylate, methylbenzyl (meth)acrylate, ethylbenzyl (meth)acrylate, propylbenzyl (meth)acrylate, methoxybenzyl (meth)acrylate, phenoxyethyl (meth)acrylate, chlorobenzyl (meth)acrylate, and nonylphenoxy polyethylene glycol (meth)acrylate.

The polyfunctional (meth)acrylate is a compound having two or more (meth) acrylate groups in the molecule, and examples thereof include a bifunctional (meth)acrylate such as neopentyl glycol di (meth)acrylate, stearic acid modified pentaerythritol di(meth)acrylate, dicyclopentenyl diacrylate, di(meth)acryloyl isocyanurate, alkylene oxide modified bisphenol di(meth)acrylate, 2,2-bis(4-(meth)acryloxyphenyl)propane, 2,2-bis(4-(meth)acryloxyethoxyphenyl)propane, 2,2-bis(4-(meth)acryloxydiethoxyphenyl)propane, 2,2-bis(4-(meth)acryloxypropoxyphenyl)propane, 2,2-bis(4-(meth)acryloxytetraethoxyphenyl)propane, and 2,2-bis(4-(meth) acryloxypolyethoxyphenyl) propane; a trifunctional or higher functional (meth)acrylate such as trimethylolpropane tri(meth)acrylate, pentaerythritol tri(meth)acrylate, tris(acryloyloxyethyl)isocyanurate, ditrimethylolpropane tetra(meth)acrylate, pentaerythritol tetra(meth)acrylate, dipentaerythritol monohydroxypenta(meth)acrylate, alkyl-modified dipentaerythritol pentaacrylate, dipentaerythritol hexa(meth)acrylate; (meth)acrylate having a polybutadiene skeleton, (meth)acrylate having a hydrogenated polybutadiene skeleton, (meth)acrylate having a polycarbonate skeleton, (meth)acrylate having a polyether skeleton, (meth)acrylate having a polyester skeleton, (meth)acrylate having a castor oil skeleton, isoprene (meth)acrylate, hydrogenated isoprene (meth)acrylate, epoxy (meth)acrylate, and (meth)acrylate having a polyphenylene ether skeleton.

Examples of the (meth)acrylate oligomer include urethane (meth)acrylate having a polybutadiene skeleton, urethane (meth)acrylate having a hydrogenated polybutadiene skeleton, urethane (meth)acrylate having a polycarbonate skeleton, urethane (meth)acrylate having a polyether skeleton, urethane (meth)acrylate having a polyester skeleton, urethane (meth)acrylate having a castor oil skeleton, isoprene-based (meth)acrylate, hydrogenated-based isoprene (meth)acrylate, epoxy (meth)acrylate, and (meth)acrylate oligomer having a polyphenylene ether skeleton.

### 8) Vinyl-based resin (hereinafter also referred to as "(B-8) component")

Examples of the vinyl compounds include: styrene and the like; a vinyl compound having two or more vinyl groups in the molecule (polyfunctional vinyl compound) such as a trialkenyl isocyanurate compound including triallyl isocyanurate (TAIC), divinylbenzene, divinylbiphenyl, divinylnaphthalene, a modified polyphenylene ether resin containing vinyl groups at both ends, polybutadiene in which the total amount of 1 and 2 bonds is 50% or more relative to the total amount of all carbon-carbon double bonds, and a styrene-butadiene copolymer having polybutadiene in which the total amount of 1 and 2 bonds is 50% or more relative to the total amount of all carbon-carbon double bonds; and a vinylbenzyl compound having two or more vinylbenzyl groups in the molecule such as a modified polyphenylene ether resin containing vinylbenzyl groups at both ends, polydivinylbenzene, a polydivinylbenzene resin, and a divinylbenzene-styrene copolymer. As the vinyl compounds, the above-described compound having two or more unsaturated double bonds in the molecule and the compound having one unsaturated double bond in the molecule may be used in combination. Specific examples of the compound having one unsaturated double bond in the molecule include a compound having one vinyl group in the molecule (monovinyl compound).

The thermosetting resin as the (B) component is preferably at least one selected from (B-3) to (B-5).

### (Crosslinking agent)

When at least one selected from the above (B-2) to (B-8) is used as the thermosetting resin as the (B) component, the resin composition of the present invention may further include a crosslinking agent. The crosslinking agent reacts with unsaturated carbon bonds included in the (B) component to form three-dimensional crosslinking.

Examples of the crosslinking agent include a polyfunctional vinyl compound such as divinylbenzene, divinylnaphthalene, and divinylbiphenyl; a vinylbenzyl ether compound synthesized from the reaction of phenol and vinylbenzyl chloride; an allyl ether compound synthesized from the reaction of styrene monomer, phenol, and allyl chloride; a trialkenyl isocyanurate such as triallyl isocyanurate (TAIC (registered trademark)) and triallyl cyanurate (TAC); a (meth)acrylate compound such as trimethylolpropane (methacrylate compound and acrylate compound); and a compound having an acenaphthylene skeleton. Using these crosslinking agents may improve heat resistance. These crosslinking agents may be used alone, or may be used by combination of two or more thereof.

As the compound having an acenaphthylene skeleton, acenaphthylene, a hydroxyacenaphthylene compound such as 3-hydroxyacenaphthylene, 4-hydroxyacenaphthylene, 5-hydroxyacenaphthylene, and 5,6-dihydroxyacenaphthylene; an alkylacenaphthylene compound such as 3-methylacenaphthylene, 3-ethylacenaphthylene, 3-propylacenaphthylene, 4-methylacenaphthylene, 4-ethylacenaphthylene, 4-propylacenaphthylene, 5-methylacenaphthylene, 5-ethylacenaphthylene, 5-propylacenaphthylene, 3,8-dimethylacenaphthylene, and 5,6-dimethylacenaphthylene; an alkoxyacenaphthylene compound such as 3-methoxyacenaphthylene, 3-ethoxyacenaphthylene, 3-butoxyacenaphthylene, 4-methoxyacenaphthylene, 4-ethoxyacenaphthylene, 4-butoxyacenaphthylene, 5-methoxyacenaphthylene, 5-ethoxyacenaphthylene, and 5-butoxyacenaphthylene; and a halogenated acenaphthylene compound such as 3-chloroacenaphthylene, 3-bromoacenaphthylene, 4-chloroacenaphthylene, 4-bromoacenaphthylene, 5-chloroacenaphthylene, and 5-bromoacenaphthylene are exemplified.

### (Polymerization initiator)

When at least one selected from (B-2) to (B-8) is used as the thermosetting resin as the (B) component, the resin composition of the present invention may contain a polymerization initiator.

The polymerization initiator used in the resin composition of the present invention include an azo-based polymerization initiator, a peroxide-based polymerization initiator, or the like.

As the azo polymerization initiator, azobisisobutyronitrile, 1,1'-azobis(cyclohexane-1-carbonitrile), 2,2'-azobis{2-methyl-N-[1,1-bis(hydroxymethyl)ethyl]propionamide}, 2,2'-azobis[2-methyl-N-(2-hydroxyethyl)propionamide], 2,2'-azobis[2-(hydroxymethyl)propionitrile], 2,2'-azobis(2,4-dimethylvaleronitrile), 2,2'-azobis(4-methoxy-2,4-dimethylvaleronitrile), dimethyl 2,2'-azobisisobutyrate, 2,2'-azobis[2-(2-imidazolin-2-yl)propane], 2,2'-azobis{2-methyl-N-[1,1-bis(hydroxymethyl)-2-hydroxyethyl]propionamide},and the like may be exemplified.

As the peroxide-based polymerization initiator, there is a peroxide such as benzoyl peroxide, cumene hydroperoxide, 2,5-dimethylhexane-2,5-dihydroperoxide, 2,5-dimethyl-2,5-di(t-butylperoxy)hexyne-3, di-t-butyl peroxide, t-butylcumyl peroxide, α,α'-bis(t-butylperoxy-m-isopropyl)benzene, 2,5-dimethyl-2,5-di(t-butylperoxy)hexane, dicumyl peroxide, di-t-butyl peroxyisophthalate, t-butyl peroxybenzoate, 2,2-bis(t-butylperoxy)butane, 2,2-bis(t-butylperoxy)octane, 2,5-dimethyl-2,5-di(benzoylperoxy)hexane, di(trimethylsilyl) peroxide, trimethylsilyltriphenylsilyl peroxide, and diisopropylbenzene hydroperoxide, but are not limited thereto. Although not a peroxide, 2,3-dimethyl-2,3-diphenylbutane may also be used as a radical polymerization initiator (or polymerization catalyst). However, the polymerization initiator used for curing the resin composition of the present invention is not limited to these examples.

The polymerization initiator used in the resin composition of the present invention is preferably a peroxide-based polymerization initiator.

### (Composition ratio of resin composition)

When the (B-1) epoxy resin is used as the (B) component, the content of the (A) component in the resin composition of the present invention is not particularly limited, but 1 to 99% by weight, 1 to 50% by weight, and 1 to 30% by weight relative to the amount of the (B) component may be exemplified.

When at least one or more selected from (B-2) to (B-8) is used as the (B) component, the content of the (A) component in the resin composition of the present invention is not particularly limited, but 1 to 99% by weight, 10 to 70% by weight, 10 to 50% by weight relative to the combined amount of the (B) component and the crosslinking agent may be exemplified.

The content ratio of the (B) component and the crosslinking agent in the resin composition of the present invention is not particularly limited, but (B) component:crosslinking agent = 99:1 to 1:99, 90:10 to 30:70, and 90:10 to 50:50 in a weight ratio may be exemplified.

In addition, the content of the polymerization initiator in the resin composition of the present invention is not particularly limited, but the amount of 0.1 to 10% by weight relative to the combined amount of the (B) component and the crosslinking agent may be exemplified.

### (2) Other components

The resin composition of the present invention may contain other components as necessary within a range that does not impair the effects of the present invention. As other components, an organic solvent, a thermoplastic resin, an inorganic filler, an organic filler, a flame retardant, and another additive may be exemplified.

### 1) Organic solvent

As the organic solvent, amide-based, ether-based, ester-based, aliphatic hydrocarbon-based, aromatic hydrocarbon-based, ketone-based, organic halogen compound-based organic solvents, and the like are exemplified.

As the amide-based organic solvent, N,N-dimethylformamide (DMF), N,N-dimethylacetamide; an ether-based organic solvent such as diethyl ether, dipropyl ether, dibutyl ether, diamyl ether, and tetrahydrofuran; an ester-based organic solvent such as ethyl acetate, propyl acetate, butyl acetate, amyl acetate, heptyl acetate, ethyl butyrate, isoamyl isovalerate, and propylene glycol methyl ether acetate; an aliphatic hydrocarbon-based organic solvent such as normal hexane, normal heptane, and cyclohexane; an aromatic hydrocarbon-based organic solvent such as toluene and xylene; a ketone-based organic solvent such as methyl ethyl ketone, methyl isobutyl ketone, and cyclohexanone; an organic halide-based organic solvent such as trichloroethane and trichloroethylene; and the like are exemplified. Further, a relatively inert organic solvent such as propylene glycol monomethyl ether and propylene glycol monoethyl ether may also be used.

### 2) Thermoplastic resin

As the thermoplastic resin, a polyacrylate resin, a polymethacrylate resin, a polystyrene resin, a polyphenylene ether resin, a polyetherimide resin, a polyether sulfone resin, a polyphenylene sulfide resin, a polycyclopentadiene resin, a polycycloolefin resin, a polycycloolefin copolymer resin, a polyarylate resin, a polyether resin, a phenoxy resin, a polyvinyl acetal resin, a polyolefin resin, a polybutadiene resin, a polyimide resin, a polyamideimide resin, a polyetherimide resin, a polysulfone resin, a polyethersulfone resin, a polycarbonate resin, a polyetheretherketone resin, a polyester resin, a liquid crystal polyester resin, and a fluororesin; a known thermoplastic elastomer, for example, a styrene-ethylene-propylene copolymer, a styrene-ethylenebutylene copolymer, a styrene-butadiene copolymer, a styrene-isoprene copolymer, a hydrogenated styrene-butadiene copolymer, a hydrogenated styrene-isoprene copolymer, a (meth)acrylonitrile-butadiene-(meth)acrylic acid copolymer, a (meth)acrylonitrile-butadiene-methyl(meth)acrylate copolymer, and a methyl (meth)acrylate-butadiene-styrene copolymer (MBS); or (meth)acrylonitrile-butadiene rubber (NBR), linear polyurethane, polybutadiene, polyisoprene, fluorine rubber, and ethylene-propylene-diene rubber may be exemplified.

### 3) Inorganic filler

The material of the inorganic filler is not particularly limited, but for example, silica, alumina, glass, cordierite, silicon oxide, barium sulfate, barium carbonate, talc, clay, mica powder, zinc oxide, hydrotalcite, boehmite, aluminum hydroxide, magnesium hydroxide, calcium carbonate, magnesium carbonate, magnesium oxide, boron nitride, aluminum nitride, manganese nitride, aluminum borate, strontium carbonate, strontium titanate, calcium titanate, magnesium titanate, bismuth titanate, titanium oxide, zirconium oxide, barium titanate, barium zirconate titanate, barium zirconate, calcium zirconate, zirconium phosphate, and zirconium tungstate phosphate are exemplified. Among these, silica is particularly suitable. In addition, as the silica, spherical silica is preferable. These inorganic fillers may be used alone, or may be used by combination of two or more thereof.

The inorganic filler is preferably treated with one or more surface treating agents such as an aminosilane-based coupling agents, an epoxysilane-based coupling agents, a mercaptosilane-based coupling agents, a silane-based coupling agents, an organosilazane compound, a titanate coupling-based agents, a vinylsilane coupling-based agent, a methacryloxysilane-based coupling agent, an acryloxysilane-based coupling agent, and styrylsilane-based coupling agent.

### 4) Organic filler

As the organic filler, rubber particles, fluororesin particles (fluoropolymer particles), polyamide fine particles, silicone particles, and the like are exemplified.

As the rubber particles, commercially available products may be used, and for example, "EXL-2655" manufactured by Dow Chemical Japan Co., Ltd. and "AC3816N" manufactured by Aica Kogyo Co., Ltd. are exemplified.

As fluororesin particles (fluoropolymer particles), for example, polytetrafluoroethylene (PTFE), perfluoroalkoxyalkane (PFA), perfluoroethylenepropene copolymer (FEP), ethylene-tetrafluoroethylene copolymer (ETFE), tetrafluoroethylene-perfluorodioxole copolymer (TFE/PDD), polyvinylidene fluoride (PVDF), polychlorotrifluoroethylene (PCTFE), ethylene-chlorotrifluoroethylene copolymer (ECTFE), and polyvinyl fluoride (PVF) are exemplified. These resins may be used alone or used by combination of two or more thereof.

As the fluororesin particles (fluoropolymer particles), commercially available products may be used. As specific examples of PTFE particles, which are commercially available fluoropolymer particles, "Lubron (registered trademark) L-2" manufactured by Daikin Industries, Ltd., "Lubron L-5" manufactured by Daikin Industries, Ltd., "Lubron L-5F" manufactured by Daikin Industries, Ltd., "Fluon (registered trademark) PTFE L-170JE" manufactured by Asahi Glass Co., Ltd., "FluonPTFE L-172JE" manufactured by Asahi Glass Co., Ltd., "FluonPTFE L-173JE" manufactured by Asahi Glass Co., Ltd., "KTL-500F" manufactured by Kitamura Co., Ltd., "KTL-2N" manufactured by Kitamura Co., Ltd., "KTL-1N" manufactured by Kitamura Co., Ltd., "TLP10F-1" manufactured by Mitsui DuPont Fluorochemical Co., Ltd., and the like are exemplified.

Surface-treated particles may be included. As the surface treatment, for example, surface treatment with a surface treating agent is exemplified. The surface treating agent is not particularly limited. The surface treating agent includes a surfactant such as a nonionic surfactant, an amphoteric surfactant, a cationic surfactant, and an anionic surfactant, as well as inorganic fine particles. From the viewpoint of affinity, it is preferable to use a fluorine-based surfactant as the surface treating agent. As specific examples of the fluorine-based surfactant, "Surflon (registered trademark) S-243" (perfluoroalkyl ethylene oxide adduct) manufactured by AGC Seimi Chemical Co., Ltd., "Megafac (registered trademark) F-251" manufactured by DIC Corporation, "Megafac F-477" manufactured by DIC Corporation, "Megafac F-553" manufactured by DIC Corporation, "Megafac R-40" manufactured by DIC Corporation, "Megafac R-43" manufactured by DIC Corporation, "Megafac R-94" manufactured by DIC Corporation, "FTX-218" manufactured by Neos Co., Ltd., "Futergent (registered trademark) 610FM" manufactured by Neos Co., Ltd., and "Futergent 730LM" manufactured by Neos Co., Ltd. are exemplified.

### 5) Flame retardant

The flame retardant is not particularly limited, but any known flame retardant may be used.

Specifically, in a field where a halogen-based flame retardant such as a brominated flame retardant is used, for example, ethylene dipentabromobenzene, ethylene bistetrabromoimide, decabromodiphenyl oxide, and tetradecabromodiphenoxybenzene, which have a melting point of 300°C or more, are preferable. It is considered that using the halogen-based flame retardant may suppress desorption of halogen at high temperatures, and may suppress a decrease in heat resistance. In addition, in a field where halogen-free is required, a phosphorus-based flame retardant such as a phosphate ester-based flame retardant, a phosphazene-based flame retardant, and a phosphinate-based flame retardant may be exemplified. As a specific example of the phosphoric acid ester-based flame retardant, a condensed phosphoric acid ester of dixylenyl phosphate is exemplified. As a specific example of the phosphazene-based flame retardant, phenoxyphosphazene is exemplified. As a specific example of the phosphinate-based flame retardant, for example, a phosphinate metal salt of a dialkyl phosphinate aluminum salt is exemplified. The exemplified flame retardants may be used alone, or may be used by combination of two or more thereof.

### 6) Other additives

As other additives, for example, an antifoaming agent such as a silicone antifoaming agent and an acrylic ester antifoaming agent; an organometallic compound such as an organocopper compound, an organozinc compound, and an organocobalt compound; an adhesion aid such as a leveling agent and a silane coupling agent; a tackiness agent such as a tackifier; a storage stabilizer such as a heat stabilizer, an antistatic agent, and BHT; an antioxidant, a light stabilizer, an ultraviolet absorber, a dye and pigment, a lubricant, a wetting and dispersing agent, a heavy metal deactivator, an ion trapping agent, an emulsifier, a water dispersion stabilizer, a mold release agent, a wax, a rheology control agent, and a surfactant are exemplified.

### (3) Aspect of using resin composition

The resin composition of the present invention may be used in a wide range of applications where the resin composition is required, such as a solder resist, an underfill material, a die bonding material, a semiconductor encapsulation material, a hole filling resin, a component embedding resin, and an insulation material for printed wiring boards. The resin composition of the present invention is preferably a resin composition for an insulating layer of printed wiring boards. As the printed wiring board, a single-sided printed circuit board, a double-sided printed circuit board, a multilayer printed circuit board, a flexible printed circuit board, a build-up circuit board, and the like are exemplified. In addition, the form of the resin composition of the present invention is not particularly limited, but the resin composition may be applied to a sheet-like laminated material such as an adhesive film and prepregs, and a printed wiring board. The resin composition of the present invention may be applied in a varnish state to a printed wiring board to form an insulating layer, but industrially, the resin composition is generally used to form an insulating layer in the form of a sheet-like laminated material such as an adhesive film or prepreg.

### 3. Cured product (molded body)

The cured product obtained by curing the resin composition of the present invention may be used as a molded body, an insulator for a printed wiring board, a metal foil-attached laminate, a cast product, an adhesive, a coating, and a film. For example, the cured product of the semiconductor encapsulation material is a cast or molded body, and as a method for obtaining a cured product for such uses, the curable resin composition is molded with a cast molding machine, a transfer molding machine, an injection molding machine, or the like, and is further heated at 80 to 230°C for 0.5 to 10 hours, allowing to provide a cured product.

### 4. Resin varnish

The resin composition of the present invention may be prepared in the form of a varnish to provide a resin varnish for being impregnated in a base material (fibrous base material) for forming prepreg, for coating a support film to form an adhesive film, or for forming a printed wiring board.

This resin varnish is particularly suitable for a printed wiring board and may be used as a varnish for a printed wiring board.

The resin varnish is prepared, for example, as follows.

Each component is charged into an organic solvent and dissolved. In this case, heating may be performed as necessary. Thereafter, as necessary, a component that does not dissolve in an organic solvent such as an inorganic filler is added and dispersed using a ball mill, bead mill, planetary mixer, roll mill, or the like to prepare a varnish-like curable resin composition.

A more excellent material for electronic parts, especially as a material for high-frequency electronic parts is provided by blending inorganic high dielectric powder such as barium titanate or inorganic magnetic material such as ferrite into a curable resin composition or resin varnish.

### 5. Sheet-like laminated material

The resin composition of the present invention may be used for a sheet-like laminated material. As the sheet-like laminated material, a prepreg, an adhesive film, and the like are exemplified.

### 6. Prepreg

The prepreg of the present invention has a base material impregnated with the resin composition of the present invention.

As the base material, a fibrous base material such as glass cloth, aramid cloth, polyester cloth, glass nonwoven fabric, aramid nonwoven fabric, polyester nonwoven fabric, pulp paper, and linter paper are exemplified.

The prepreg of the present invention may be produced by a known method. For example, there is exemplified a method in which a base material is impregnated with the resin varnish of the present invention and then dried.

The base material impregnated with the resin varnish is heated under desired heating conditions, for example, at 80 to 170°C for 1 to 10 minutes to remove the solvent, allowing to provide a prepreg in a semi-cured state (B stage).

### 7. Adhesive film

The adhesive film of the present invention has a resin composition layer containing the resin composition of the present invention on a support film. The adhesive film of the present invention may be produced by a known method. For example, the adhesive film may be produced by applying the resin varnish of the present invention to a support film using a dicoder or the like, further drying the organic solvent by heating or blowing hot air to form a resin composition layer.

The drying conditions are not particularly limited, but drying is performed such that the content of the organic solvent in the resin composition layer becomes 10% by mass or less, preferably 5% by mass or less. Although depending on the amount of the organic solvent in the varnish and the boiling point of the organic solvent, for example, a varnish containing 30 to 60% by mass of an organic solvent is dried at 50 to 150°C for about 3 to 10 minutes, allowing to form a resin composition layer.

The thickness of the resin composition layer formed in the adhesive film is preferably more than or equal to the thickness of the conductor layer. The thickness of the conductor layer included in a circuit board is typically in the range of 5 to 70 µm, and thus the resin composition layer preferably has a thickness of 10 to 100 um. From the viewpoint of thinning the film, the thickness is more preferably 15 to 80 µm.

As the supporting film, various plastic films such as a polyolefin film including polyethylene, polypropylene, and polyvinyl chloride; a polyester film such as polyethylene terephthalate (hereinafter sometimes abbreviated as "PET") and polyethylene naphthalate; a polycarbonate film, and a polyimide film are exemplified. In addition, release paper or metal foil such as copper foil, and aluminum foil may be used. Among these, from the viewpoint of versatility, a plastic film is preferable, and the polyethylene terephthalate film is more preferable. The support and the protective film described below may be subjected to the surface treatment such as mud treatment and corona treatment. In addition, a mold release treatment may be performed with a mold release agent such as a silicone resin-based mold release agent, an alkyd resin-based mold release agent, and a fluororesin-based mold release agent.

The thickness of the support film is not particularly limited, but is preferably 10 to 150 um, more preferably 25 to 50 µm.

A protective film similar to the support may be further laminated on the surface with which the support of the resin composition layer is not in close contact. The thickness of the protective film is not particularly limited, but is, for example, 1 to 40 µm. Laminating the protective film may prevent dust and the like from adhering to or scratching the surface of the resin composition layer. The adhesive film may also be wound up into a roll and stored.

### 8. Insulator for printed wiring boards

The insulator for printed wiring boards of the present invention includes a cured product of either the prepreg of the present invention or the adhesive film of the present invention. The method for producing the insulator for printed wiring boards of the present invention is not particularly limited, but for example, a method of stacking one or more sheet-like laminate materials and molding them under heat and pressure is exemplified. The insulator for printed wiring boards of the present invention is suitable as an insulating layer for single-sided printed circuit boards, double-sided printed circuit boards, multilayer printed circuit boards, flexible printed circuit boards, and build-up circuit boards.

### 9. Metal foil-attached laminate

The metal foil-attached laminate of the present invention includes a layer of the insulator for printed wiring boards of the present invention and a layer of metal foil. As the metal foil used herein, copper foil, aluminum foil, and the like are exemplified. The thickness is not particularly limited, but is in the range of 3 to 200 µm, more preferably 3 to 105 µm.

As the method for producing the metal foil-attached laminate of the present invention, for example, a method of laminating the above-described sheet-like laminated material of the present invention and metal foil in a layer configuration according to the purpose, bonding the layers each other under heat and pressure, and simultaneously curing with heat may be exemplified. In the metal foil-attached laminate of the present invention, the insulator for printed wiring boards and the metal foil are laminated in an optional layer configuration. The metal foil may be used both as a surface layer and as an intermediate layer. In addition, it is possible to repeat lamination and curing multiple times to form a multilayer structure.

An adhesive may also be used for adhesion to the metal foil. As the adhesive, epoxy-based, acrylic-based, phenol-based, cyanoacrylate-based adhesives, and the like are exemplified, but are not particularly limited thereto. In addition, the resin composition of the present invention may be used as an adhesive. The above lamination molding and curing may be performed under the same conditions as those for producing the insulator for printed wiring boards of the present invention.

### Industrial Applicability

The resin composition containing the polymer of the present invention may be processed into a molded material, sheet, or film, and may be used for a low dielectric material, an insulating material, a heat-resistant material, a structural material, and the like that may satisfy properties such as low dielectric constant, low water absorption, and high heat resistance in fields such as the electrical industry, space aircraft industry, and automobiles. In particular, the resin composition may be used as a single-sided, double-sided, and multilayer printed circuit board, a flexible printed circuit board, a build-up circuit board, and the like. Further, the resin composition may be applied to a semiconductor-related material or optical material as well as a paint, a photosensitive material, an adhesive, a sewage treatment agent, a heavy metal scavenger, an ion exchange resin, an antistatic agent, an antioxidant, an antifogging agent, an antirust agent, an anti-dye agent, a fungicide, an insect repellent, a medical material, a flocculant, a surfactant, a lubricant, a binder for solid fuel, a conductive treatment agent, a resin modifier, an asphalt modifier plasticizer, a sintered binder, and the like.

The polymer of the present invention is a resin that has a low dielectric constant and a low dielectric loss tangent, has a high glass transition point, and exhibits high solubility in an organic solvent and high compatibility with a thermosetting resin. Therefore, in fields such as the electrical and electronic industry, and the space aircraft industry, as the dielectric material, insulating material, heat-resistant material, structural material, and the like, in response to the recent strong demand for downsizing and thinning, it is possible to provide a cured molded body that is free from the molding defect such as warping. Further, due to the excellent wiring embedding flatness and the excellent adhesion with different materials, it is possible to achieve a curable resin composition, a cured product, or a material including the same with excellent reliability.

Examples are shown below, but the technical scope of the invention of the present application is not limited to Examples.

### Examples

### (1) Weight average molecular weight (Mw) and dispersity (Mw/Mn)

The weight average molecular weight of the polymer obtained in Examples was measured using the following device and conditions.

### [Device]

Sample injection device: Waters 2695 Alliance
Separation column: ShodexKF-G, 805L, 804L, 804L
Detector: Waters 2414 differential refraction (RI) detector 2998 Photodiode array (PDA) detector
Column oven: Waters column oven

### [Conditions]

Column oven temperature: 40°C
RI detector temperature: 40°C
Mobile phase: tetrahydrofuran
Flow rate: 1.0 mL/min
Standard injection volume: 200 µL
PDA detector extraction wave: 254.0 nm
Quantitative calculation: Standard polymethyl methacrylate conversion

### (2) Glass transition temperature

Using 10 mg of polyacrylamide as a sample, using a DSC (differential scanning calorimetry) device (TA Instruments Q2000), the temperature was raised at a temperature increase rate of 10 °C/min, and the intermediate value between the two bending point temperatures derived from the glass transition in the temperature increase curve was defined as the glass transition temperature.

### (3) Relative dielectric constant and dielectric loss tangent

Measurement was performed using a resonance method with polyacrylamide powder under the following conditions. Device: Cylindrical cavity resonator manufactured by AET Inc.
Frequency: 1 GHz
Measurement temperature: 23°C

### (4) Solubility

An organic solvent having 50 parts by weight was added to 50 parts by weight of polyacrylamide, and the mixture was stirred at room temperature for 2 hours. The state of the resin solution after one day of standing was evaluated based on the criteria below. MEK, toluene, and cyclohexanone were used as an organic solvent.
Good: The resin solution had fluidity and was transparent.
Fair: The resin solution had fluidity but was cloudy.
Bad: The resin solution had no fluidity, and the resin was not dissolved at all.

### (Example 1)

### Synthesis of N,N-bis(4-(tert-butyl)phenyl)acrylamide

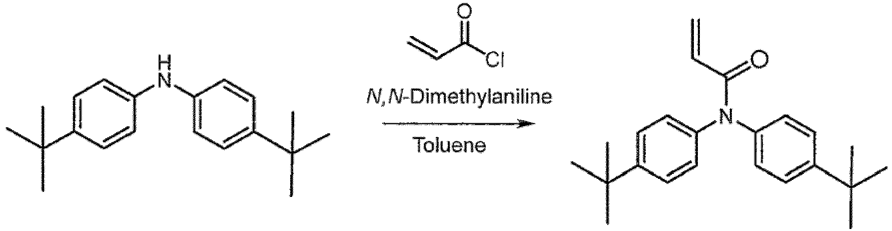

In a 1 L four-necked flask purged with nitrogen, bis(4-tert-butylphenyl)amine (100 g, 0.355 mol), N,N-dimethylaniline (64.59 g, 0.533 mol), and 400 mL of super-dehydrated toluene were charged and stirred until uniformly dissolved. Then, the reaction solution was cooled to 0°C or less in an ice/ethanol bath, and acrylic acid chloride (38.59 g, 0.426 mol) was slowly added dropwise thereto, followed by stirring for 30 minutes. Thereafter, the temperature of the reaction solution was raised to room temperature, and the reaction was performed for 24 hours. After the reaction was completed, washing was performed with a 1N aqueous hydrochloric acid solution, a saturated aqueous sodium bicarbonate solution, and a saline solution. The organic layer was dehydrated with magnesium sulfate, and then the filtrate was distilled off using an evaporator. The obtained crude product was purified by recrystallization with hexane to obtain N,N-bis(4-(tert-butyl)phenyl)acrylamide (92.90 g, yield 79%).

### (Example 2)

### Preparation of polyacrylamide A: poly[N,N-bis(4-(tert-butyl)phenyl)acrylamide]

In a 300 mL four-necked flask, 50 g of N,N-bis(4-(tert-butyl)phenyl)acrylamide and 0.15 g of AIBN were charged and dissolved in 75 g of toluene. Deaeration was performed by applying a reduced pressure operation, and the mixture was heated and stirred at 65°C for 24 hours under a nitrogen atmosphere. Then, 0.10 g of AIBN was added and stirred at 80°C for 2 hours. While heating and stirring was stopped, the reaction solution was sampled and measured by gel permeation chromatography. The reaction solution was dropped into 1 L of methanol to perform powderization. The obtained precipitate was filtered and dried in a vacuum dryer at 60°C under a reduced pressure.

### Yield 46.2 g, Mw = 58,000, Mw/Mn = 2.40

### (Comparative Example 1)

### Synthesis of N,N-bis(4-(n-butyl)phenyl)acrylamide

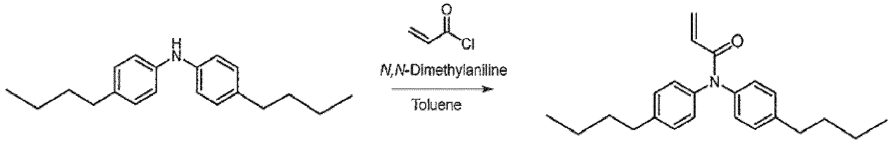

In a 300 mL four-necked flask purged with nitrogen, bis(4-n-butylphenyl)amine (25.4 g, 0.090 mol), N,N-dimethylaniline (21.09 g, 0.174 mol), and 100 mL of ultra-dehydrated toluene were charged and stirred until uniformly dissolved. Then, the reaction solution was cooled to 0°C or less in an ice/ethanol bath, and acrylic acid chloride (9.46 g, 0.105 mol) was slowly added dropwise thereto, followed by stirring for 30 minutes. Thereafter, the temperature of the reaction solution was raised to room temperature, and the reaction was performed for 24 hours. After the reaction was completed, the mixture was washed with 1N aqueous hydrochloric acid, saturated aqueous sodium bicarbonate, and distilled water. The organic layer was dehydrated with magnesium sulfate, and then the filtrate was distilled off using an evaporator. The obtained crude product was separated and purified by silica gel column chromatography using hexane/ethyl acetate = 4:1 v/v as a solvent to obtain N,N-bis(4-(n-butyl)phenyl)acrylamide (20.80 g, yield 69%).

### (Comparative Example 2)

### Preparation of polyacrylamide B: Poly[N,N-bis(4-(n-butyl)phenyl)acrylamide]

In a 100 mL two-necked flask, 20 g of N,N-bis(4-(n-butyl)phenyl)acrylamide and 0.06 g of AIBN were charged and dissolved in 22.5 g of toluene. Deaeration was performed by applying a reduced pressure operation, and the mixture was heated and stirred at 60°C for 24 hours under a nitrogen atmosphere. Then, 0.10 g of AIBN was added and the mixture was stirred at 60°C for 24 hours. While heating and stirring was stopped, the reaction solution was sampled and measured by gel permeation chromatography. The reaction solution was dropped into 1 L of methanol to perform powderization. The obtained precipitate was filtered and dried in a vacuum dryer at 60°C under a reduced pressure. Yield 18.1 g, Mw = 76,000, Mw/Mn = 2.89

**[Table 1]**

| | | Example 2 | Comparative Example 2 |
|---|---|---|---|
| Polyacrylamide | | A | B |
| Tg | °C | 252 | 128 |
| Relative dielectric constant | 1GHz | 2.41 | 2.41 |
| Dielectric loss tangent | 1GHz | 0.0035 | 0.0039 |
| Solubility | MEK | ○ | ○ |
| | Toluene | ○ | ○ |
| | Cyclohexanone | ○ | ○ |

From this test result, it has been found that the polyacrylamide of the present invention has excellent heat resistance and dielectric properties, and can be dissolved in an organic solvent at a high concentration, and has excellent compatibility with other thermoplastic resins and thermosetting resins.

### (Example 3, Comparative Example 3)

Each reagent having the composition (parts by weight) shown in Table 2 was added to toluene and mixed so that the solid content concentration became 50% by weight. Thereafter, toluene was removed from the obtained liquid using an evaporator to provide a powdered resin composition. The resin composition was cured by hot pressing at 200°C for 90 minutes. The physical properties of the obtained cured product were measured for the following items, and the results are shown in Table 2.

### <Glass transition temperature (Tg)>

Using a DSC (differential scanning calorimetry) device (Q2000 manufactured by TA Instruments, Inc.), the temperature was raised at a heating rate of 10°C/min. The intermediate value between the two bending point temperatures derived from the glass transition in the temperature increase curve was defined as the glass transition temperature.

### <Dielectric constant test (Dk), dielectric loss tangent test (Df)>

Measurement was performed at 10 GHz using a cylindrical cavity resonator (TE mode resonator) manufactured by AET Inc.

**[Table 2]**

| Run | | Example 3 | Comparative Example 3 |
|---|---|---|---|
| Composition (parts by weight) | NC3000 | 8.00 | 8.00 |
| | HPC-8000-65T | 12.00 | 12.00 |
| | DMAP | 0.02 | 0.02 |
| | Polyacrylamide A | 2.00 | - |
| *T_{g}* | °C | 158 | 150 |
| *Dₖ* | 10 GHz | 2.51 | 2.63 |
| *D_{f}* | 10 GHz | 0.0040 | 0.0045 |

| | | | |
|---|---|---|---|
| NC3000: Biphenyl type epoxy resin manufactured by Nippon Kyaku Co., Ltd. HPC-8000-65T: Active ester resin manufactured by DIC Corporation DMAP: Dimethylaminopyridine | | | |

### (Example 4, Comparative Example 4)

Each reagent having the composition (parts by weight) shown in Table 3 was added to toluene and mixed so that the solid content concentration became 50% by weight. Thereafter, toluene was removed from the obtained liquid using an evaporator to provide a powdered resin composition. The resin composition was cured by hot pressing at 200°C for 120 minutes. The physical properties of the obtained cured product were measured for the following items, and the results are shown in Table 3.

### <Glass transition temperature (Tg)>

Using a DSC (differential scanning calorimetry) device (Q2000 manufactured by TA Instruments, Inc.), the temperature was raised at a heating rate of 10°C/min. The intermediate value between the two bending point temperatures derived from the glass transition in the temperature increase curve was defined as the glass transition temperature.

### <Dielectric constant test (Dk), dielectric loss tangent test (Df)>

Measurement was performed at 10 GHz using a cylindrical cavity resonator (TE mode resonator) manufactured by AET Inc.

**[Table 3]**

| | | Example 4 | Comparative Example 4 |
|---|---|---|---|
| Composition (parts by weight) | SA9000 | 100 | 100 |
| | Polyacrylamide A | 30 | - |
| | Perbutyl P | 0.5 | 0.5 |
| Evaluation | Tg(°C) | 168 | 157 |
| | Dk | 2.45 | 2.50 |
| | Df | 0.0028 | 0.0030 |

| | | | |
|---|---|---|---|
| SA9000: Methacrylic modified polyphenylene ether compound manufactured by SABIC innovative plastics Limited Perbutyl (registered trademark) P: α,α'-di(t-butylperoxy)isopropylbenzene manufactured by NOF Corporation | | | |

## Claims

1. A polymer comprising at least one repeating unit derived from a polymerizable compound of formula (I): 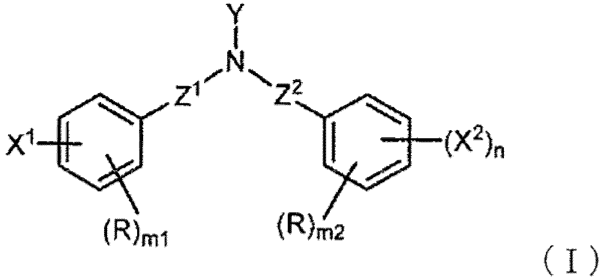 wherein X¹ and X² each independently represent a C3 to C6 branched alkyl group, a C3 to C6 cyclic alkyl group, a C3 to C6 branched alkoxy group, or a C3 to C6 cyclic alkoxy group, n represents 0 or 1, Z¹ and Z² each independently represent a single bond or a C1 to C3 alkylene group, each R independently represents an organic group or a halogeno group, m1 and m2 each independently represent any integer of 0 to 4, and Y represents a polymerizable functional group.

2. The polymer according to claim 1, wherein Y is an acryloyl group or a methacryloyl group.

3. A resin composition comprising the polymer according to claim 1.

4. The resin composition according to claim 3, further comprising a resin other than the polymer according to claim 1.

5. The resin composition according to claim 4, wherein the resin other than the polymer according to claim 1 is a thermosetting resin.

6. The resin composition according to claim 5, wherein the thermosetting resin is an epoxy resin.

7. The resin composition according to claim 6, further comprising an active ester-based compound as a curing agent.

8. The resin composition according to claim 5, wherein the thermosetting resin is at least one or more selected from:
a maleimide compound having one or more maleimide groups;
a polyphenylene ether compound;
a polybutadiene having a molar ratio of 1,2-bond structure to 1,4-bond structure of 80:20 to 100:0;
a styrene-butadiene-styrene block copolymer (SBS) in which a molar ratio of 1,2-bond structure to 1,4-bond structure in a butadiene block is 80:20 to 100:0; and
a polymer having, in a molecule, a repeating unit of formula (V) : 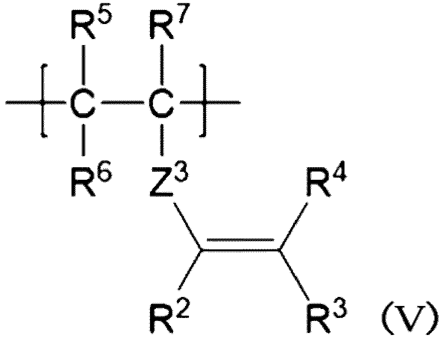
wherein Z³ represents a C6 to C12 arylene group, R² to R⁷ each independently represent a hydrogen atom or a C1 to C6 alkyl group.

9. The resin composition according to claim 8, wherein a polyphenylene ether compound is a polyphenylene ether compound terminal-modified with a group of formula (VI), an acryloyl group, or a methacryloyl group, 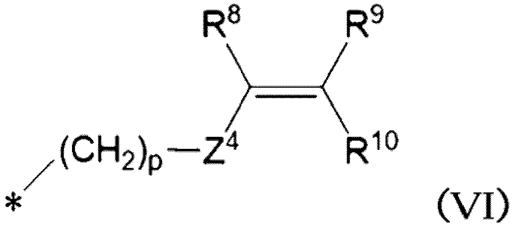 wherein, in formula (VI), * represents a bonding position, p represents an integer of 0 to 10, Z⁴ represents a C6 to C12 arylene group, and R⁸ to R¹⁰ each independently represent a hydrogen atom or a C1 to C6 alkyl group.

10. A molded body comprising a cured product of the resin composition according to claim 3.

11. The resin composition according to any one of claims 3 to 9, which is a resin composition for an insulating layer of printed wiring boards.

12. A resin varnish comprising the resin composition according to any one of claims 3 to 9.

13. A prepreg comprising a base material impregnated with the resin composition according to any one of claims 3 to 9.

14. An adhesive film comprising a resin composition layer containing the resin composition according to any one of claims 3 to 9 on a support film.

15. An insulator for printed wiring boards, consisting of a cured product of the prepreg according to claim 13.

16. An insulator for printed wiring boards, consisting of a cured product of the adhesive film according to claim 14.

17. A metal foil-attached laminate, comprising:
a layer consisting of the insulator for printed wiring boards according to claim 15; and
a layer consisting of metal foil.

18. A metal foil-attached laminate, comprising a layer consisting of the insulator for printed wiring boards according to claim 16 and a layer consisting of metal foil.

19. A compound of formula (III): 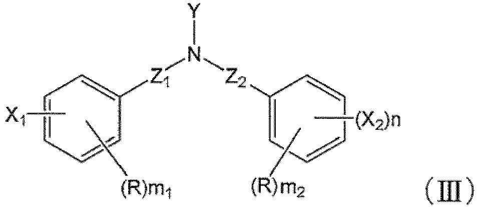 wherein X¹ and X² each independently represent a C3 to C6 branched alkyl group, a C3 to C6 cyclic alkyl group, a C3 to C6 branched alkoxy group, or a C3 to C6 cyclic alkoxy group, n represents 0 or 1, Z¹ and Z² each independently represent a single bond or a C1 to C3 alkylene group, each R independently represents an organic group or a halogeno group, m1 and m2 each independently represent any integer of 0 to 4, and Y represents a polymerizable functional group.

20. The compound according to claim 19, wherein the compound of formula (III) is a compound of formula (IV): 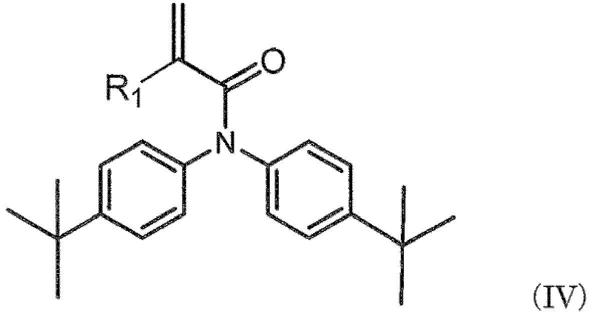 wherein R₁ represents a hydrogen atom or a methyl group.
